# EUROPEAN PATENT APPLICATION

(11) **EP 1 944 366 A1**
(43) Date of publication of application: **16.07.2008**
(21) Application number: 06810828.1
(22) Date of filing: 29.09.2006
(51) Int. Cl.: C12N 15/09, C12N 9/88, C12P 13/00

(54) **NOVEL ACID-RESISTANT MUTANT S-HYDROXYNITRILE LYASE**

(30) Priority: 29.09.2005 JP 2005285049
(71) Applicant: Nippon Shokubai Co.,Ltd., Osaka-shi, Osaka 541-0043 (JP); National Institute of Technology and Evaluation, Tokyo 151-0066 (JP)
(72) Inventor: ICHIGE, Eita, Chikusei-shi Ibaraki 308-0847 (JP); SEMBA, Hisashi, Tsuchiura-shi Ibaraki 300-0810 (JP); SHIJUKU, Toshiaki, Kisarazu-shi Chiba 920-814 (JP); HARAYAMA, Shigeaki, Kisarazu-shi Chiba 292-0818 (JP)
(74) Representative: Dempster, Robert Charles
(86) International application number: PCT/JP2006/319422
(87) International publication number: WO 2007/037354

(57) **Abstract**

This invention relates to a novel acid-resistant modified S-hydroxynitrile lyase (SHNL). More particularly, this invention relates to SHNL that is obtained by substituting at least one amino acid selected from amino acids 36, 140, and 209 in the amino acid sequence (SEQ ID NO: 2) of wild-type SHNL with another amino acid and has improved acid resistance compared with wild-type SHNL.

## Description

### Technical Field

The present invention relates to a novel acid-resistant modified S-hydroxynitrile lyase (SHNL). More particularly, the present invention relates to SHNL that is obtained by modifying an amino acid sequence at a specific site and has improved acid resistance compared with wild-type SHNLs.

### Background Art

S-Hydroxynitrile lyase (SHNL) is an industrially important enzyme that catalyzes the reaction between hydrocyanic acid and aldehyde or ketone to generate optically active cyanohydrins.

SHNLs derived from Cassava (*Manihot esculenta*), Pará rubber tree (*Hevea brasiliensis*), poaceous plants (i.e., sorghum (*Sorghum bicolor)),* and the like have been known. At the industrial level, recombinant SHNL is used in addition to wild-type SHNLs because separation of enzymes from organisms incurs a high cost.

Recombinant SHNL can be produced using E. *coli,* yeast, or other hosts. From an industrial point of view, use of modified SHNL with improved resistance or activity is preferable in order to further improve cost performance. In the reaction between a carbonyl compound and hydrocyanic acid that is catalyzed by SHNL, in particular, the racemization reaction that is not induced by an enzyme simultaneously proceeds. It is known that such competitive reaction is inhibited by performing the reaction under acidic conditions. If modified SHNL having acid resistance could be produced, therefore, it would be possible to produce optically active cyanohydrins in a more efficient manner.

An example of a known modified SHNL is a modified SHNL that is prepared by substituting tryptophane 128 with alanine in the amino acid sequence of SHNL to improve enzyme activity (JP Patent Publication (Kokai) No. 2000/125886 A; Lauble et al., Protein Science, 2002, 11: pp. 65-71). However, SHNL with improved acid resistance has not yet been reported.

### Disclosure of the Invention

An object of the present invention is to provide novel SHNL with significantly improved acid resistance compared with wild-type SHNL to realize more efficient production of optically active cyanohydrin.

The present inventors have conducted concentrated studies in order to attain the above object. As a result, they found that amino acid substitution in SHNL through genetic engineering could produce enzymes having significantly improved acid resistance compared with the enzyme before modification. This has led to the completion of the present invention.

More specifically, the present invention relates to modified SHNL, which is obtained by substituting at least one amino acid selected from amino acids 36, 140, and 209 in the amino acid sequence (SEQ ID NO: 2) of wild-type SHNL derived from cassava *(Manihot esculenta)* with another amino acid and to modified SHNL, which is obtained by substituting at least one amino acid selected from amino acids 36, 139, and 208 in the amino acid sequence (SEQ ID NO: 3) of wild-type SHNL derived from the Pará rubber tree *(Hevea brasiliensis)* with another amino acid.

A specific example of such modified SHNL is one comprising at least one amino acid substitution selected from the amino acid substitutions shown below in the amino acid sequence (SEQ ID NO: 2) of wild-type SHNL derived from cassava (*Manihot esculenta*)*:*
a) substitution of leucine 36 with methionine;
b) substitution of threonine 140 with isoleucine; and
c) substitution of lysine 209 with asparagine.

The aforementioned modified SHNL may further comprise at least one amino acid substitution selected from the following amino acid substitutions:
a) substitution of lysine 21 with aspartic acid, glutamic acid, or asparagine;
b) substitution of glycine 165 with aspartic acid or glutamic acid;
c) substitution of valine 173 with leucine;
d) substitution of methionine 174 with leucine; and
e) substitution of threonine 163 with aspartic acid, glutamic acid, or serine.

The present invention also provides DNA that encodes the amino acid sequence of the modified SHNL.

The present invention provides a method for producing modified SHNL comprising culturing a host cell into which the aforementioned DNA has been introduced and recovering a protein having SHNL activity from the resulting culture product.

Further, the present invention provides a method for producing optically active cyanohydrin comprising allowing the modified SHNL of the present invention to react with a carbonyl compound and cyanide.

The acid-resistant modified SHNL of the present invention has significantly improved acid resistance compared with existing enzymes. Accordingly, such enzymes can undergo reactions under acidic conditions, and efficient production of optically active cyanohydrin can be realized while suppressing competitive racemization.

### Brief Description of the Drawings

Fig. 1 is an alignment diagram showing amino acid sequences of SHNL derived from cassava *(Manihot esculenta)* and SHNL derived from the Pará rubber tree (*Hevea brasiliensis).*
Fig. 2 shows the results of evaluating acid resistance of a mutant.
Fig. 3 shows the results of evaluating acid resistance of a mutant with combination of two or more mutation sites.
Fig. 4 shows the results of evaluating heat resistance of an acid-resistant modified SHNL.
Fig. 5 shows the results of evaluating solvent resistance of an acid-resistant modified SHNL (A: ethanol resistance; B: ethyl acetate resistance).
Fig. 6 shows the results of an attempt to improve heat resistance of a gene of a heat-resistant modified enzyme by newly adding a site of acid-resistant mutation.
Fig. 7 is a graph showing the results of comparing thermostability of wild-SHNL and that of Actmt-001f2-SHNL.
Fig. 8 is a photograph showing the results of analyzing the heated wild-SHNL and Actmt-001f2-SHNL samples via SDS-PAGE.

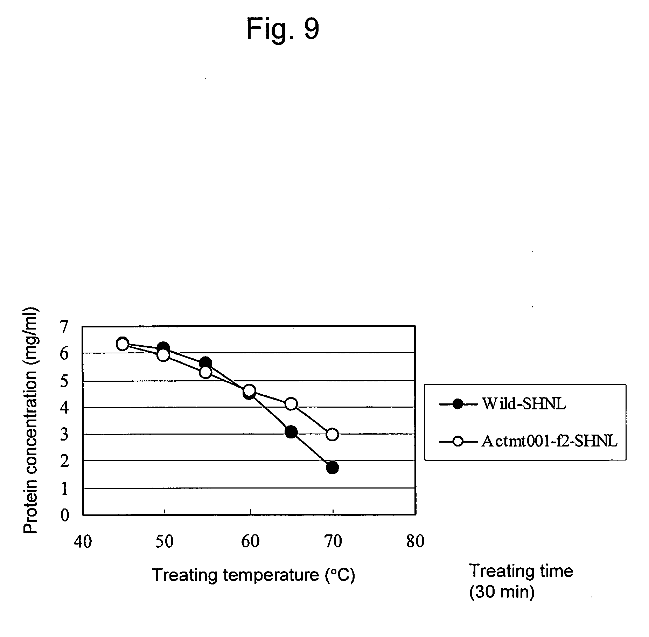

Fig. 9 is a graph showing changes in protein levels in the heated wild-SHNL and Actmt-001f2-SHNL samples.

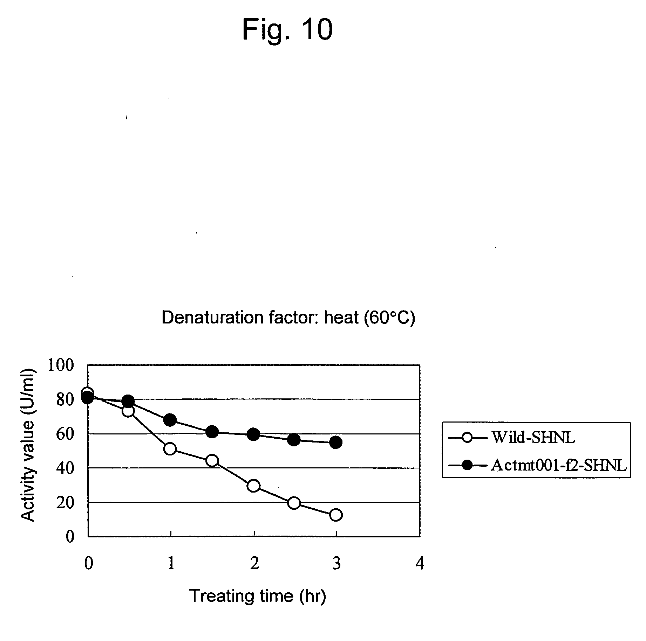

Fig. 10 is a graph showing the results of comparing thermostability of wild-SHNL and that of Actmt-001f2-SHNL.
Fig. 11 is a photograph showing the results of analyzing the heated wild-SHNL and Actmt-001f2-SHNL samples (supernatants) via SDS-PAGE.
Fig. 12 is a graph showing organic solvent resistance of Actmt-001f2-SHNL (A: ethanol resistance; B: ethyl acetate resistance).
Fig. 13 is a graph showing the optical purity of S-mandelonitrile 1 hour after the initiation of the repetitive reaction of Actmt-001f2-SHNL.
Fig. 14 is a graph showing the rate of benzaldehyde conversion through repetitive reactions of Actmt-001f2-SHNL (A: changes in the conversion rate 1 hour after the initiation of the reaction depending on the number of repetitions; B: changes in the conversion rate in the 11 th reaction with the elapse of time).
Fig. 15 is a graph showing changes in enzyme activities of various types of modified SHNL via heating.
Fig. 16 is a graph showing thermostability of modified enzyme T163S-SHNL.
Fig. 17 is a graph showing the results of comparing thermostability of wild-SHNL, that of Actmt-001f2-SHNL, and that of V173L-SHNL.
Fig. 18 is a graph showing organic solvent resistance of V173L-SHNL (A: ethanol resistance; B: ethyl acetate resistance).
Fig. 19 is a graph showing the thermostability of modified enzyme Actmt0020-b8-SHNL.
Fig. 20 is a graph showing the thermostability of Lys-21 modified enzyme.
Fig. 21 is a graph showing the thermostability of SHNL having multiple modification sites.
Fig. 22 is a graph showing ethanol resistance of G 165E,V173L-SHNL.
Fig. 23 is a graph showing ethyl acetate resistance of G165E,V173L,M174L-SHNL.
Fig. 24 is a graph showing the amount of 2CMN produced 1 hour after the initiation of repetitive reactions using G165E, V173L-SHNL.
Fig 25 is a graph showing the optical purity of S-2CMN obtained by the first repetition of synthesis under each pH condition.
Fig. 26 is a graph showing the optical purity of S-2CMN obtained by the fourth repetition of synthesis under each pH condition.

This description includes part or all of the contents as disclosed in the description of Japanese Patent Application No. 2005-285049, which is a priority document of the present application.

### Best Modes for Carrying out the Invention

Hereafter, the present invention is described in detail.

### 1. Wild-type S-hydroxynitrile lyase

In the present invention, the term "wild-type S-hydroxynitrile lyase (hereafter abbreviated to "SHNL")" refers to SHNL isolated and purified from a plant or SHNL having an amino acid sequence identical to that of the former SHNL. The origin of wild-type SHNL is not particularly limited. Examples thereof include SHNL derived from poaceous plants such as sorghum *(Sorghum bicolor*), SHNL derived from Euphorbiaceae plants such as cassava *(Manihot esculenta)* or Pará rubber tree (*Hevea brasiliensis),* and SHNL derived from Olacaceae plants such as *Ximenia amerieana.* The amino acid sequences and the nucleotide sequences of the genes of such SHNLs are already known and can be easily obtained from public databases such as GenBank. For example, the SHNL gene derived from the Pará rubber tree *(Hevea brasiliensis*), the SHNL gene derived from *Manihot esculenta,* and the SHNL gene derived from sorghum are registered in GenBank under the accession nos. U40402 (SEQ ID NO: 3 is equivalent to CDS of U40402), Z29091, and AJ421152, respectively.

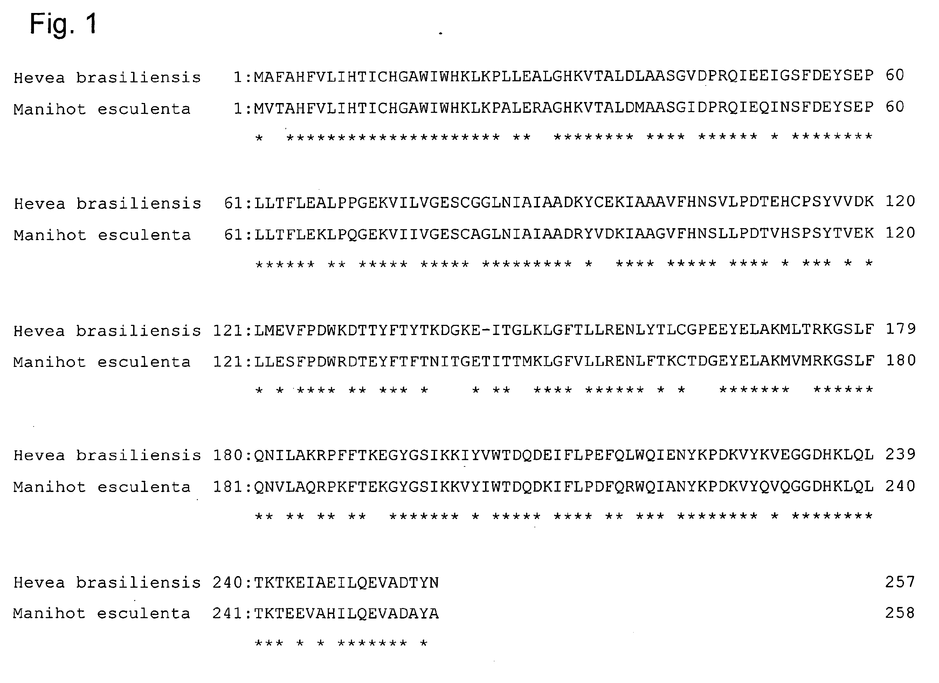

Fig. 1 shows the aligned amino acid sequences of the cassava *(Manihot esculenta*)-derived SHNL and of the Pará rubber tree *(Hevea brasiliensis)-derived* SHNL. Amino acid homology between these SHNLs is 74%, and amino acids of each SHNL are not completely identical. For example, *Hevea brasiliensis*-derived SHNL lacks the amino acid that is equivalent to amino acid 139 of *Manihot esculenta-derived* SHNL. Thus, the amino acid number in the helix D3' domain is out of alignment by one residue. Specifically, amino acids 36, 140, and 209 in the amino acid sequence of SHNL derived from cassava (SEQ ID NO: 2) are equivalent to amino acids 36, 139, and 208 in the amino acid sequence of SHNL derived from the Pará rubber tree (*Hevea brasiliensis*) (SEQ ID NO: 4).

SHNLs derived from cassava and derived from the Pará rubber tree belong to the α/β hydrolase superfamily and their conformations are very similar to each other. Based on the effects of modification of an amino acid sequence by SHNL derived from cassava, accordingly, similar effects can be expected concerning SHNL derived from the Pará rubber tree *(Hevea brasiliensis)* by modification of an amino acid sequence at a relevant site.

### 2. Modified S-hydroxynitrile lyase

The present invention relates to acid-resistant modified SHNL, which is obtained by modification (i.e., substitution or insertion) of an amino acid sequence at a given site of an amino acid sequence of wild-type SHNL derived from cassava or from Pará rubber tree. Specifically, the present invention relates to modified SHNL, which is obtained by substituting at least one amino acid selected from amino acids 36, 140, and 209 in the amino acid sequence (SEQ ID NO: 2) of wild-type SHNL derived from cassava *(Manihot esculenta)* with another amino acid and to modified SHNL, which is obtained by substituting at least one amino acid selected from amino acids 36, 139, and 208 in the amino acid sequence (SEQ ID NO: 4) of wild-type SHNL derived from the Pará rubber tree *(Hevea brasiliensis)* with another amino acid.

A more specific example of modified SHNL is one comprising at least one amino acid substitution selected from the amino acid substitutions shown below in the amino acid sequence (SEQ ID NO: 2):
a) substitution of leucine 36 with methionine;
b) substitution of threonine 140 with isoleucine; and
c) substitution of lysine 209 with asparagine.

Further, SHNL that additionally comprises the above-mentioned modification site has higher acid resistance. For example, modified SHNL comprising the aforementioned amino acid substitutions at positions 36 and 140 or the aforementioned amino acid substitutions at positions 36, 140, and 209 added to the amino acid sequence of wild-type SHNL derived from cassava (SEQ ID NO: 2) has higher acid resistance.

Further, modified SHNL comprising amino acid substitution at one or more sites selected from modification sites 21, 163, 165, 169, 172, 173, and 174 for improving heat resistance, which have been already reported by the present inventors, has high acid resistance in combination with high heat resistance. Specifically, the modified SHNL of the present invention has high acid resistance in combination with high heat resistance when it further comprises at least one amino acid substitution for improving heat resistance selected from:
a) substitution of lysine with aspartic acid, glutamic acid, or asparagine at position 21;
b) substitution of glycine 165 with aspartic acid or glutamic acid;
c) substitution of valine 173 with;
d) substitution of methionine 174 with leucine; and
e) substitution of threonine 163 with aspartic acid, glutamic acid, or serine.

Amino acid substitution and insertion may be carried out via site-directed mutagenesis into a gene that encodes the amino acid sequence in accordance with a conventional technique. Such site-directed mutagenesis can be easily performed using a commercially available kit (for example, QuikChange XL Site-Directed Mutagenesis Kit (Stratagene) or Transformer^{™} Site-Directed Mutagenesis Kit (Clontech.)). Since the modified SHNL of the present invention has improved acid resistance compared with wild-type SHNL, the reaction can be performed under acidic conditions while suppressing racemization. Thus, the modified SHNL of the present invention can be a very useful enzyme for industrial production of optically active cyanohydrin.

### 3. Production of acid-resistant modified S-hydroxynitrile lyase

### 3.1 DNA that encodes acid-resistant modified SHNL

DNA that encodes the modified SHNL protein according to the present invention is obtained by introducing site-directed mutations into the gene of known wild-type SHNL. Specifically, a pair of primers that can modify a codon at a substitution site into a codon that encodes the amino acid of interest is designed. Next, the resulting pair of primers is used to perform an extension reaction utilizing DNA that encodes wild-type SHNL as a template. Site-directed mutagenesis can be easily carried out utilizing commercialized kits (for example, QuikChange XL Site-Directed Mutagenesis Kit (Stratagene) or Transformer^{™} Site-Directed Mutagenesis Kit (Clontech)).

### 3.2 Recombinant vector

DNA that encodes the aforementioned acid-resistant modified SHNL is then ligated (inserted) to (into) a known vector such as a plasmid to prepare a recombinant vector. Such vector is not particularly limited as long as it can replicate the gene of interest in a host. Examples include plasmid DNA and phage DNA.

Examples of such plasmid DNA include *E.* coli-derived plasmids (for example, a pET21 vector having a particularly potent T7 promoter, such as pBR322, pBR325, pUC18, pUC119, pHCEIIB, pTrcHis, or pBlueBacHis, is preferable), *Bacillus subtilis-derived* plasmids (for example, pUB110 and pTP5), and yeast-derived plasmids (for example, YEp13, YEp24, YCp50, and pYE52). An example of phage DNA is λphage.

The gene of the present invention is inserted into the aforementioned vector by first cleaving purified DNA with an adequate restriction enzyme and inserting the cleaved DNA into an adequate restriction enzyme site or multi-cloning site of the vector DNA for ligation.

In order to express a foreign gene in a host, an adequate promoter needs to be positioned prior to the positioning of a structural gene. Such promoter is not particularly limited. Any promoter that is known to function in a host can be employed. Promoters will be described in detail concerning each host in sections concerning transformants below. If necessary, a cis element such as an enhancer, splicing signal, poly A additional signal, libosome binding sequence (SD sequence), terminator sequence, and the like may be positioned.

### 3.3 Modified SHNL expression system (transformant)

Subsequently, the aforementioned recombinant vector is introduced into a host in a manner such that the target gene can be expressed therein to prepare a modified SHNL expression system. A host cell is not particularly limited as long as the DNA of the present invention can be expressed therein. Examples thereof include: bacteria belonging to *Escherichia* such as *Escherichia coli, Bacillus* such as *Bacillus subtilis, Pseudomonas* such as *Pseudomonas putida,* or *Rhizobium* such as *Rhizobium meliloti;* yeast such as *Saccharomyces cervisiae, Schizosaccharomyces pombe,* and *Pichia pastoris;* animal cells such as COS cells and CHO cells; and insect cells such as Sf19 and Sf21.

When a bacterium such as *E. coli* is used as a host, it is preferable that the recombinant vector of the present invention be capable of self-replicating in the bacterium and, at the same time, also be comprised of a promoter, a ribosome binding sequence, the gene of the present invention, and a transcription termination sequence. Further, it may also comprise a gene for regulating a promoter. Examples of E. *coli* include *Escherichia coli* HMS174 (DE3), K12, DH1, and B strains, and examples of *Bacillus subtilis* include *Bacillus subtilis* MI 114 and 207-21. A promoter is not particularly limited as long as it can express the gene of interest in a host such as *E. coli.* For example, E. coli-derived or phage-derived promoters can be employed, such as: trp promoter, lac promoter, P_{L} promoter, and P_{R} promoter. Alternatively, an artificially designed and modified promoter, such as a tac promoter, may also be employed. A method for introducing a recombinant vector into a bacterium is not particularly limited. For example, a method involving the use of calcium ions (Cohen, S. N. et al., Ploc. Natl. Acad. Sci., U.S.A., 69: 2110-2114, 1972) and electroporation can be employed.

Where yeast is used as a host, for example, *Saccharomyces cerevisiae, Schizosaccharomyces pombe,* or *Pichea pastris,* is used. A promoter is not particularly limited as long as it can express the gene of interest in yeast. For example, gal1 promoter, gal10 promoter, heat shock protein promoter, MFα1 promoter, PHO5 promoter, PGK promoter, GAP promoter, ADH promoter, or AOX1 promoter can be employed. A method for introducing a vector into yeast is not particularly limited. Examples of such methods include electroporation (Becker, D. M. et al., Methods. Enzymol., 194: 182-187, 1990), the spheroplast method (Hinnen, A. et al., Proc. Natl. Acad. Sci., U.S.A., 75: 1929-1933, 1978), and the lithium acetate method (Itoh, H., J. Bacteriol., 153: 163-168, 1983).

### 3.4 Culture of transformant

The modified SHNL of the present invention can be obtained by culturing the transformant according to the present invention in an adequate medium and recovering a protein having enzyme activity from the culture product. A method for culturing the transformant according to the present invention is adequately determined in accordance with the host. In the case of a transformant where a microorganism such as E. *coli* or yeast is employed as a host, for example, either a natural or synthetic medium may be used as long as it contains carbon sources, nitrogen sources, and inorganic salts assimilable by the microorganism and is capable of efficiently culturing the transformant.

During the culture, an antibiotic such as ampicillin or tetracycline may be added to the medium, if necessary. When a microorganism transformed with an expression vector containing an inducible promoter is cultured, an inducer may be added to the medium, if necessary. When a microorganism transformed with an expression vector containing lac promoter is cultured, for example, isopropyl-β-thiogalactopyranoside (IPTG) may be added to the medium. When culturing a microorganism transformed with an expression vector containing trp promoter, indoleacrylic acid (IAA) or the like may be added to the medium.

If the enzyme protein of the present invention is produced in the relevant microorganism or cell after the culture, the cultured microorganism or cell is homogenized. If the protein of the present invention is secreted outside of the microorganism or cell, the culture broth may be used in such state or may be subjected to centrifugation or another procedure to remove the microorganism or cell.

Ammonium sulfate precipitation, SDS-PAGE, gel filtration, ion exchange chromatography, affinity chromatography, or other means are employed independently or in an appropriate combination to isolate and purify proteins.

The enzyme activity of the modified SHNL according to the present invention can be confirmed by adding the enzyme to a reaction solution that contains a substrate, i.e., adequate cyanide, and aldehyde or ketone and detecting the generated optically active cyanohydrin. Optically active cyanohydrin can be confirmed by, for example, gas chromatography or high-performance liquid chromatography. Alternatively, an antibody that specifically binds to the modified SHNL of the present invention can be prepared to confirm the expression via Western blotting using the resulting antibody. For example, the enzyme activity of SHNL can be confirmed by assaying the amount of aldehyde generated per unit time (calculated based on absorbance at 249.6 nm) upon degradation of mandelonitrile by SHNL.

The modified SHNL of the present invention can be produced in accordance with the methods disclosed in JP Patent Publication (Kokai) Nos. 10-373246 A (1998), 10-373248 A (1998), or 11-367251 A (1999).

### 4. Synthesis of optically active cyanohydrin using acid-resistant modified SHNL

The modified SHNL of the present invention can synthesize optically active cyanohydrin with production efficiency and optical purity higher than those of wild-type SHNL. Optically active cyanohydrin can be synthesized with the use of the present invention's acid-resistant modified SHNL in the same manner as wild-type SHNL.

Specifically, the modified SHNL of the present invention and a reaction substrate are added to a reaction solvent, and the reaction is carried out at 10°C to 50°C for 20 minutes to 24 hours. Thus, optically active cyanohydrin can be synthesized. The reaction time is adequately determined in accordance with the conversion rate of the substrate. Examples of a reaction substrate that can be employed include a carbonyl compound and cyanide. A carbonyl compound is an aldehyde or ketone represented by COR1R2 wherein R1 and R2 each independently represent: a hydrogen atom, substituted or non-substituted, linear or branched, and saturated alkyl having 1 to 18 carbon atoms, or a substituted or non-substituted cyclic 5-22-membered aromatic group, provided that R1 and R2 do not simultaneously represent a hydrogen atom. Cyanide is not particularly limited as long as it generates cyanide ions (CN⁻). Examples thereof that can be employed include hydrogen cyanides such as sodium cyanide or potassium cyanide and cyanohydrins such as acetone cyanohydrin.

Use of a reaction solvent mainly composed of an organic solvent that is hardly soluble or insoluble in water is preferable from the viewpoint described below. That is, when a large quantity of water is present in a reaction system, racemization of optically active cyanohydrin generated via enzyme reaction is likely to occur. When aldehyde or ketone having a low degree of water solubility is used as a starting material, the production efficiency deteriorates. Such organic solvent is not particularly limited if it does not affect the synthesis of optically active cyanohydrin via enzyme reaction. An organic solvent can be adequately selected in accordance with properties of aldehyde or ketone used as a starting material for synthesis or properties of cyanohydrin, which is a generated product. Specific examples include: aliphatic or aromatic, linear, branched, or cyclic, and saturated or unsaturated hydrocarbon solvents that may be optionally halogenated, such as pentane, hexane, toluene, xylene, and methylene chloride; aliphatic or aromatic, linear, branched, or cyclic, and saturated or unsaturated alcohol solvents that may be optionally halogenated, such as isopropyl alcohol, n-butanol, isobutanol, t-butanol, hexanol, cyclohexanol, and n-amyl alcohol; aliphatic or aromatic, linear, branched, or cyclic, and saturated or unsaturated ether solvents that may be optionally halogenated, such as diethyl ether, dipropyl ether, diisopropyl ether, dibutyl ether, and methyl t-butyl ether; and aliphatic or aromatic, linear, branched, or cyclic, and saturated or unsaturated ester solvents that may be optionally halogenated, such as methyl formate, methyl acetate, ethyl acetate, butyl acetate, and methyl propionate. They may be used alone or in combinations of two or more. An aforementioned solvent that comprises or is saturated with water or an aqueous buffer can also be employed.

In the process of industrial production, the modified SHNL may be utilized as an enzyme immobilized on an adequate inorganic carrier (see, for example, JP Patent Publication (Kokai) No. 2002-176974 A). Examples of preferable methods for synthesizing cyanohydrin with the use of the modified SHNL of the present invention include those disclosed in JP Patent Publication (Kokai) Nos. 2002-355085 A, 2002-176974 A, 2001-363840 A, 2001-346596 A, 2001-190275 A, 2000-245286 A, 2001-120289 A, and 2000-217590 A.

### Examples

Hereafter, the present invention is described in greater detail with reference to examples and reference examples, although the technical scope of the present invention is not limited thereto.

### Example 1

### Example 1: Screening for of acid-resistant mutant SHNL from library of random mutant SHNL

### [Material and method]

### 1) Library of random mutant SHNL

The SHNL gene used in the present invention comprises a sequence (SEQ ID NO: 1: JP Patent Application No. 2002-365675 (hereafter this SHNL is referred to as "wild-SHNL") resulting from conversion of the gene sequence of SHNL cloned from cassava (*Manihot esculenta*) into an *E. coli*-type codon. With the use of a plasmid vector, SHNL-Wild/pET21a comprising the gene of the aforementioned wild-SHNL introduced into a pET21a vector (Novagen) as a template, mutation was introduced randomly using the GeneMorph^{™}PCR Mutagenesis Kit (Stratagene) to prepare a library of random mutant SHNLs. The resulting library of random mutant SHNL was inserted into a multicloning site of a plasmid vector, pKK223-3 (Amersham Biosciences), transformed into *Escherichia coli* DH5α, and then cryopreserved in that state.

### 2) Screening for of acid-resistant mutant SHNL

Clones obtained from the library of random mutant SHNL were inoculated on a deep-well plate into which NS-2 medium shown in Table 1 had been dispensed, and shake culture was carried out at 20°C and 1,100 rpm.

**Table 1**

| Composition of NS-2 medium | |
|---|---|
| Glycerol | 40 g |
| (NH₄)₂SO₄ | 10 g |
| KH₂PO₄ | 2 g |
| K₂HPO₄ | 6 g |
| Yeast ext. (Ebios P2G) | 40 g |
| MgSO₄·7H₂O | 1 g |
| Ampicillin | 1 g |
| IPTG | 0.238g |
| Distilled water | 1 liter |

After the cells had grown sufficiently, the culture solution was centrifuged to obtain a cell pellet. The cell pellet was suspended in 150 µl of sodium citrate buffer (pH 5.5), and the cells were then homogenized using Shake Master (BMS) to obtain a crude enzyme solution. Subsequently, 10 µl of the crude enzyme solution was added to 150 µl of sodium citrate buffer (pH 4.15), and the mixture was then subjected to acid treatment with shaking at 1,100 rpm and 20°C for 2 hours. To the crude enzyme solution after the acid treatment, 150 µl of sodium citrate buffer (pH 4.15) was then added, 0.04 µl of a substrate, i.e., DL-mandelonitrile, was further added, and an enzyme reaction was carried out with shaking. The reaction was terminated by adding 30 µl of phosphoric acid 20 minutes later, the reaction solution was transferred to a UV plate, the absorbance at 260 nm was assayed using a plate reader (GENios, TECAN), and the obtained value was designated as an activity value. Subsequently, a crude enzyme solution that had been subjected to shaking in a sodium citrate buffer (pH 5.5) at 1,100 rpm and 20°C for 2 hours was used as a control to perform an enzyme reaction at pH 5.5, and the activity value was also assayed. The activity value of the acid-treated enzyme solution was divided by that of the control, and the obtained value was used as an indicator for acid resistance. As a reference, the crude enzyme solution prepared from a culture solution of SHNL-Wild/pKK223-3/DH5α was subjected to the same assay to determine the indicator for acid resistance of wild-SHNL. A mutant exhibiting an indicator for higher acid resistance compared with wild-SHNL was selected as an acid-resistant mutant. The selected acid-resistant mutant was subjected to plasmid extraction, and sequence analysis was carried out using the extracted plasmid as a template to identify a mutation site.

### 3) Evaluation of acid resistance of the selected mutant

The selected acid-resistant enzyme strain and SHNL-Wild/pKK223-3/DH5α as a control were cultured in a test tube, and a crude enzyme solution was prepared by homogenizing the culture solution after the completion of culture. A sodium citrate buffer (pH 5.5) and an E. coli-derived inactive protein were added to the resulting crude enzyme solution, the activity value of the enzyme solution was adjusted to 2 U/ml, and a protein concentration thereof was adjusted to 1 mg/ml.

The aforementioned E. *coli* strain was cultured using 5 ml of NS-2 medium shown in Table 1. Culture was conducted with agitation and shaking at 20°C and 120 rpm. The SHNL enzyme activity was determined by assaying, using DL-mandelonitrile as a substrate, the rate of benzaldehyde generation, which is generated by degrading DL-mandelonitrile by an enzyme based on changes in the absorbance at 249.6 nm. The protein concentration was assayed using the BCA protein assay kit (Pierce) and BSA as a standard. The E. coli-derived inactive protein was obtained by homogenizing a culture solution of the E. *coli* strain pKK223-3/DH5α.

Subsequently, 150 µl of a citrate buffer (pH 4.15) was added to 30 µl of the prepared crude enzyme solution, and the resultant was subjected to acid treatment with agitation at 20°C for 2 hours. The activity was assayed using the acid-treated crude enzyme solution, and the activity remaining after the reaction was determined by designating the activity before acid treatment as 100%.

### [Results]

Table 2 shows the sites of mutation in the selected acid-resistant mutant SHNL strain. In the selected acid-resistant mutants, Lot002H6 and Lot034B10, cytosine 106 in the SHNL gene sequence as shown in SEQ ID NO: 1 mutated into adenine. As a result, leucine 36 of the SHNL amino acid sequence as shown in SEQ ID NO: 2 mutated into methionine. In the Lot023F12 strain, similarly, cytosine 419 in the SHNL gene sequence as shown in SEQ ID NO: 1 mutated into thymine, and threonine 140 in the SHNL amino acid sequence as shown in SEQ ID NO: 2 consequently mutated into isoleucine. In the Lot016G12 strain, adenine 627 in the SHNL gene sequence as shown in SEQ ID NO: 1 mutated into thymine, and lysine 209 in the SHNL amino acid sequence as shown in SEQ ID NO: 2 consequently mutated into asparagine.

**Table 2: Mutation site of acid-resistant mutant SHNL strains**

| Strains | Mutant amino acid no. | Before mutation | | After mutation | |
|---|---|---|---|---|---|
| | | Codon | Amino acid | Codon | Amino acid |
| Lot002H6 | 36 | CTG | Leu | ATG | Met |
| Lot034B10 | | | | | |
| Lot023F12 | 140 | ACT | Thr | ATT | Ile |
| Lot016G12 | 209 | AAA | Lys | AAT | Asn |

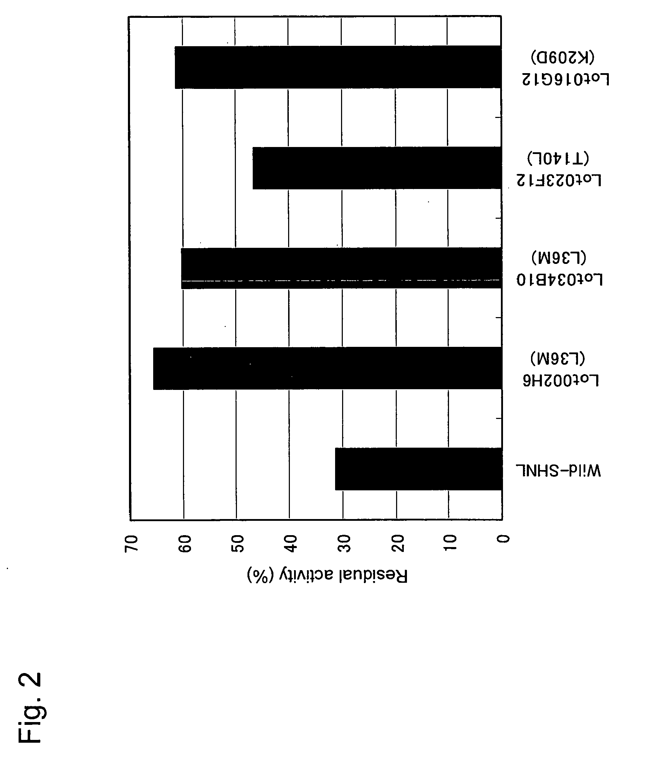

The results of acid-resistance evaluation are shown in Fig. 2. Such results demonstrated that SHNL having at least one mutation selected from among mutation of leucine 36 to methionine (hereafter referred to as "L36M"), mutation of threonine 140 to isoleucine (hereafter referred to as "T140I"), and mutation of lysine 209 to asparagine (hereafter referred to as "K209N") in the SHNL amino acid sequence (SEQ ID NO: 2) had improved acid resistance.

Hereafter, acid-resistant mutant SHNL obtained by culturing Lot002H6 or Lot034B10 is referred to as "L36M-SHNL," acid-resistant mutant SHNL obtained by culturing Lot023F12 is referred to as "T140I-SHNL," and acid-resistant mutant SHNL obtained by culturing Lot016G12 is referred to as "K209N-SHNL."

### Example 2: Improvement of acid resistance by combination of mutation sites

Acid-resistant mutant enzyme strains selected from the library of random mutant SHNLs independently had an amino acid mutation site. Thus, further improvement of acid resistance was attempted by combining such mutation sites on a single gene.

### [Material and method]

Mutation sites were combined using the QuikChange XL Site-Directed Mutagenesis Kit (Stratagene), and site-directed mutagenesis of 3 mutations, i.e., L36M, T140I, and K209N, into the SHNL amino acid sequence was carried out in various combinations. As a template, 10 ng of a plasmid vector SHNL-Wild/pKK223-3, into which the gene of wild-SHNL had been incorporated, was used.

Site-directed mutagenesis of L36M involved the use of primers as shown in SEQ ID NOs: 5 and 6 shown below. Similarly, site-directed mutagenesis of T140I involved the use of primers as shown in SEQ ID NOs: 7 and 8, and site-directed mutagenesis of K209N involved the use of primers as shown in SEQ ID NOs: 9 and 10.
SEQ ID NO: 5: GGCCACAAAGTTACTGCAATGGACATGGCAGCCAGTGGC
SEQ ID NO: 6: GCCACTGGCTGCCATGTCCATTGCAGTAACTTTGTGGCC
SEQ ID NO: 7: CACGTTCACCAACATCATCGGCGAAACCATCACTACCATG
SEQ ID NO: 8: CATGGTAGTGATGGTTTCGCCGATGATGTTGGTGAACGTG
SEQ ID NO: 9: ATTTGGACCGATCAAGACAACATATTCCTGCCGGACTTCCAACGC
SEQ ID NO: 10: GCGTTGGAAGTCCGGCAGGAATATGTTGTCTTGATCGGTCCAAAT

The resulting PCR product was transformed into a competent cell, DH5α, to prepare a recombinant *E. coli* strain comprising combined SHNL mutations.

Subsequently, the recombinant E. *coli* strain obtained in accordance with the method described in Example 1 3) was cultured, and the acid resistance of the combined mutant was evaluated using the obtained crude enzyme solution. A sodium citrate buffer (pH 5.5) and an E. coli-derived inactive protein were adequately added to bring the activity value to 43 U/ml and the protein concentration to 19.25 mg/ml of the enzyme solution. For comparison, SHNL-Wild/pKK223-3/DH5α, Lot002H6, Lot016G12, and Lot023F12 were similarly cultured, and crude enzyme solutions were prepared and then evaluated.

### [Results]

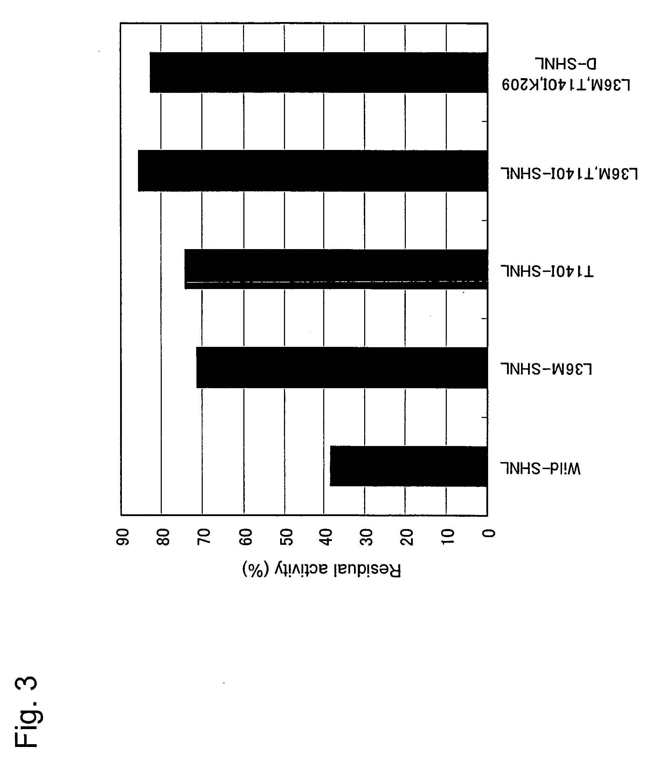

After the acid treatment, acid resistant mutant SHNL, L36M-SHNL and T140I-SHNL, independently having a mutation site exhibited the residual activity of approximately 70%. However, the L36M,T140I-SHNL strain comprising 2 mutations in combination and the L36M,T140I,K209D-SHNL strain comprising 3 mutations in combination had further improved acid resistance compared with the aforementioned acid resistant enzymes. Both the L36M,T140I-SHNL strain and the L36M,T140I,K209D-SHNL strain maintained 80% or higher activity after the acid treatment at pH 4.15 for 2 hours (Fig. 3). These results demonstrated that combination of mutation sites on a single gene enabled further improvement of acid resistance.

### Example 3: Solvent resistance of acid-resistant mutant SHNL

### [Material and method]

The DH5α/pKK223-3/SHNL-K209D strain was cultured in the same manner as in Example 1 3) to obtain an enzyme solution. A buffer and bovine serum albumin were added to the resulting enzyme solution, the activity value and the protein concentration of the enzyme solution were adjusted to given levels, the enzyme solution was treated with the use of ethanol and ethyl acetate, and the residual activity was assayed.

### [Results]

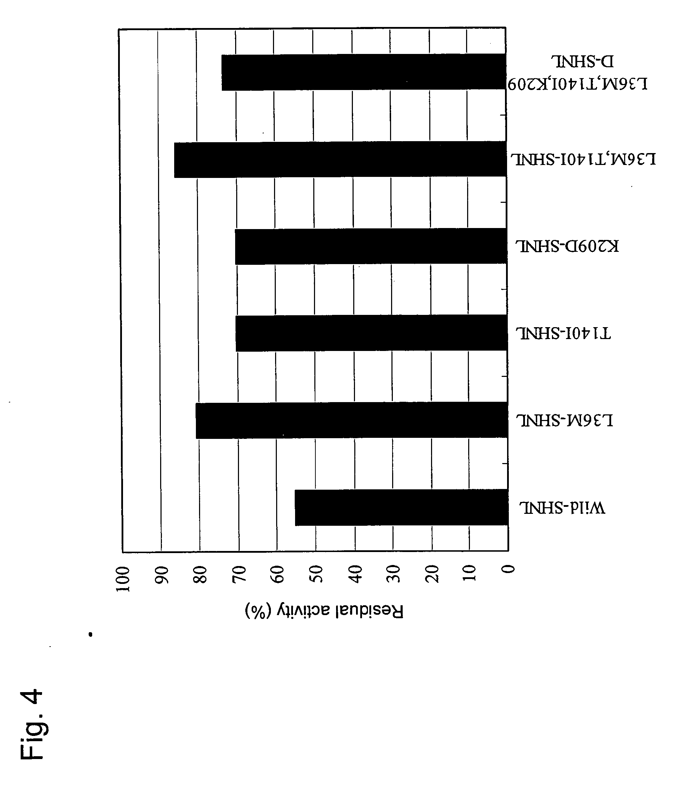

The enzyme solution was treated with ethanol and, consequently, K209D-SHNL was found to maintain 50% of its original activity after treatment of the same duration (Fig. 4). Also, K209D-SHNL exhibited high resistance to ethyl acetate, and 50% or more activity remained after 48 hours of treatment. Thus, the acid-resistant mutant SHNL was found to have organic solvent resistance.

### Example 4: Heat resistance of acid resistant mutant SHNL

### [Material and method]

The acid resistant enzyme solution prepared in Example 2 and wild-SHNL were used to heat the enzyme solution to 60°C, and the residual activity was assayed 30 minutes later.

### [Results]

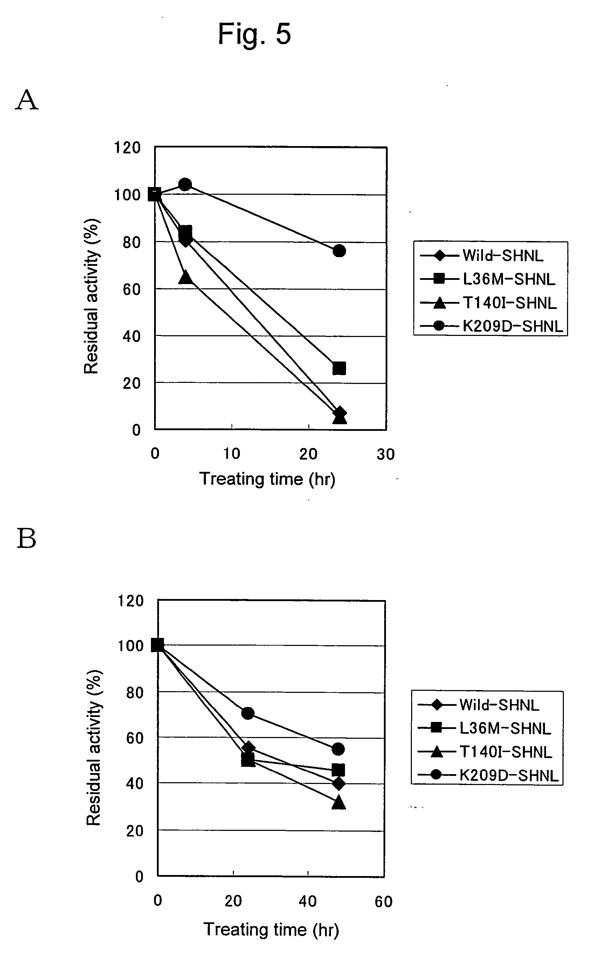

Fig. 5 shows the residual activity of acid-resistant mutant SHNLs after heat treatment. The acid resistant mutant SHNLs had improved heat resistance as well as improved acid-resistant.

### Example 5: Combination of acid-resistance mutation site and heat-resistant mutation site [Material and method]

Mutation sites were combined using the QuikChange XL Site-Directed Mutagenesis Kit (Stratagene). The SHNL-G165E,V173L/pET21a plasmid was used as a template, and 3 amino acid mutation sites were combined on the SHNL-G165E,V173L gene. The constructed combined mutant and, as controls, BL21(DE3)/pET21a/SHNL-Wild and SHNL-G165E,V173L were used to prepare enzyme solutions, and a sodium citrate buffer (pH 5.5) and an E. coli-derived inactive protein were adequately added to bring the activity value to 43 U/ml and the protein concentration to 19.25 mg/ml in the enzyme solution. Subsequently, the enzyme solution was heated to 45°C to 70°C, and 30 minutes later, the residual activity was assayed.

### [Results]

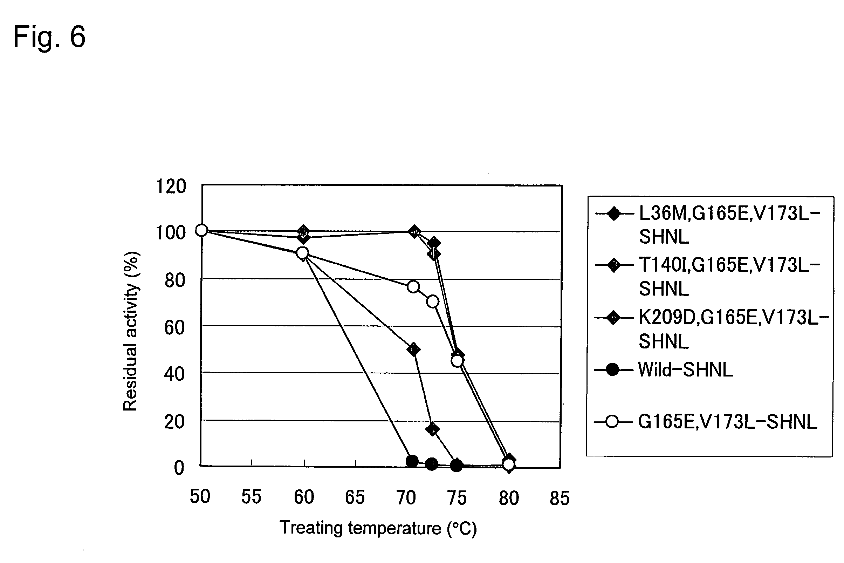

An enzyme strain comprising combined mutations, which was prepared by newly adding L36M or T140I to the heat-resistant enzyme gene comprising combined mutations, had further improved heat resistance, and 100% and 90% or higher activity thereof remained at 70°C and 72.5°C, respectively (Fig. 6).

Accordingly, heat resistance can further be improved by newly adding an acid-resistant mutation site to the heat-resistant enzyme gene comprising combined mutations.

### Example 6: Synthesis of optically active cyanohydrin using acid-resistant SHNL

The acid-resistant SHNL of the present invention has significantly improved acid resistance compared with conventional enzymes. Accordingly, such enzyme can undergo stable reactions under conditions that are more acidic than conventional conditions, and optically active cyanohydrin can be effectively synthesized while suppressing competitive racemization.

The L36M,G165E,V173L-SHNL and T140I,G165E,V173L-SHNL enzymes prepared in Example 5 into which acid-resistant mutations had been introduced were subjected to repetitive synthesis of optically active cyanohydrin at three different pH conditions, i.e., at a common pH level of 5.5 and more acidic pH levels of 4.5 and 4.1. The resultants were compared with the G165E,V173L-SHNL enzyme into which acid resistant mutation had not been introduced.

### [Material and method]

### 1) Preparation of enzyme solution

The *E. coli* strains, BL21(DE3)/pET21a/SHNL-L36M,G165E,V173L and BL21(DE3)/pET21a/SHNL-T140I,G165E,V173L, were cultured in accordance with the method described in Example 1 3). As a control, the BL21(DE3)/pET21a/SHNL-G165E,V173L strain into which acid resistant mutation had not been introduced was cultured in the same manner, and cells were recovered by centrifugation following the completion of culture. A 0.2 M sodium citrate buffer was added to the recovered cells in an amount twice that of the cells, and the cells were ultrasonically homogenized to obtain a crude enzyme solution.

Further, a 0.2 M sodium citrate buffer and an E. coli-derived inactive protein were added to the crude enzyme solutions, the activity values were adjusted to 87.4 U/ml, and the specific activity values were adjusted to 8.2 U/ml. The E. coli-derived inactive protein was obtained by homogenizing the culture solution of the *E. coli* BL21 (DE3)/pET21a strains. Silica gel (300 mg) was mixed with 0.3 ml of the crude enzyme solution to obtain immobilized enzymes.

### 2) Enzyme reaction

To t-butylmethylether (4.492 ml) comprising HCN dissolved therein to a final concentration of 1.61 M, 0.337 ml of a 0.2 M citrate buffer was added. The resulting mixture was agitated for 30 minutes and allowed to stand thereafter, and the separated aqueous phase was removed. The pH values of the 0.2 M citrate buffer used were at a common level of 5.5 and more acidic levels of 4.5 and 4.1. The solution was added to a 9-ml screw vial containing 600 mg of the immobilized enzymes prepared above. 2-Chlorobenzaldehyde (2CBA) was added to a final concentration of 1.0 M therein, and an enzyme reaction was carried out by stirring with the use of a bottle roller. The reaction solution (4 ml) was recovered 3 hours after the initiation of the reaction. Continuously, the HCN/t-butylmethylether solution treated in the same manner and an equivalent amount of 2CBA were added to carry out the enzyme reaction, 5 ml of the reaction solution was recovered 3 hours after the initiation of the reaction, and the optical purity of the synthesized S-2-chloromandelonitrile (S-2CMN) and the conversion thereof from 2CBA were determined based on the results of assaying the concentration of (R/S)-2-chloromandelonitrile in the reaction solution. The enzyme reaction was repeated 4 times in total (it should be noted that the reaction of the L36M,G165E,V173L-SHNL enzyme was carried out once at pH 5.5 and not repeated).

### [Results]

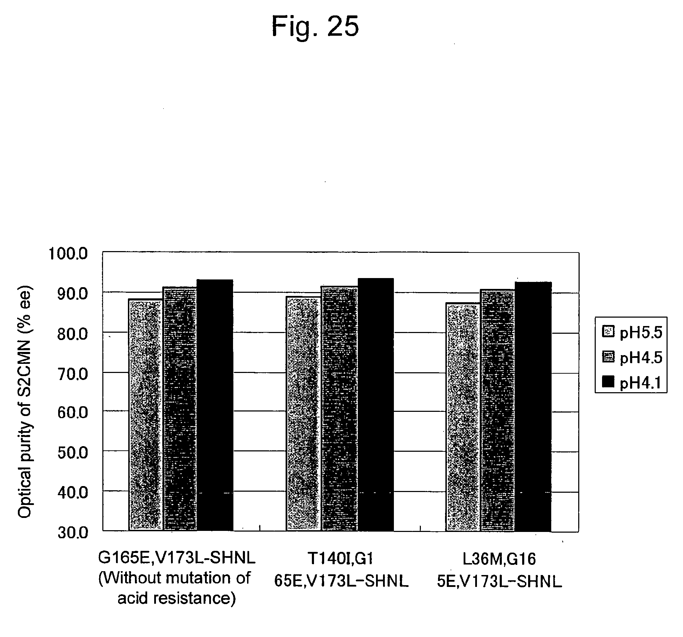

Fig. 25 shows the optical purity of S-2CMN obtained by the first repetitive synthesis under each pH condition. It was consequently demonstrated that S-2CMN of higher optical purity would be obtained as the acidity of the pH level became stronger, regardless of the presence of acid resistant mutation.

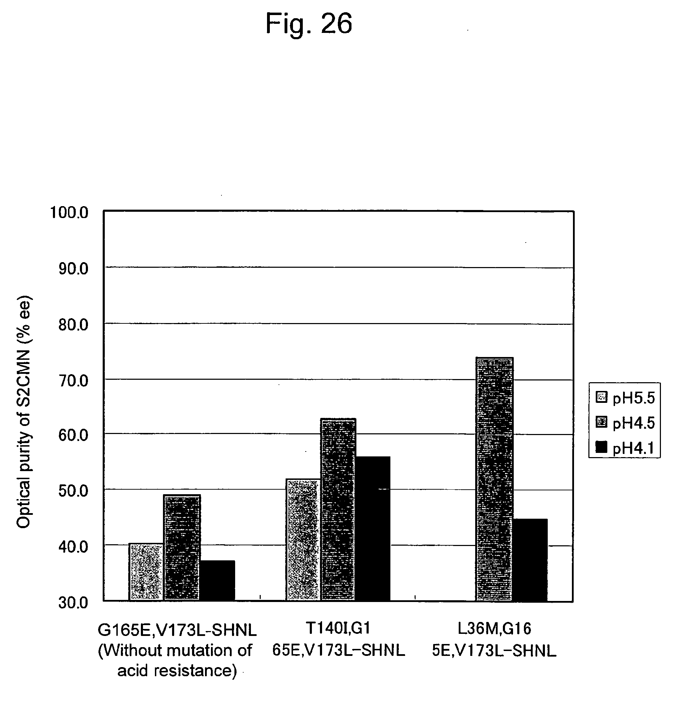

Fig. 26 shows the optical purity of S-2CMN that was obtained in the fourth repetitive synthesis reaction. The optical purity of the G165E,V173L-SHNL enzyme strain into which acid-resistant mutation had not been introduced was significantly lowered, regardless of the pH conditions. In the case of the L36M,G165E,V173L-SHNL and T140I,G165E,V173L-SHNL enzyme strains into which acid resistant mutation had been introduced, however, lowering of optical purity was mild. At pH 4.5, S-2CMN having optical purity of 63% ee was obtained from T140I,G165E,V173L-SHNL, and that of 74% ee was obtained from L36M,G165E,V173L-SHNL. These results demonstrate that an enzyme into which acid resistant mutation had been introduced would be more stable at lower pH levels compared with an enzyme into which acid resistant mutation had not been introduced and that S-2CMN of high optical purity would be produced after repeating the reactions 4 times.

Thus, use of an enzyme into which acid resistant mutation had been introduced was found to enable stable production of optically active cyanohydrin of high optical purity.

Hereafter, examples concerning modification of SHNL for improving heat resistance are demonstrated as reference examples.

### Reference Example 1: Preparation of mutant enzyme Actmt-001f2-SHNL

### 1. Mutagenesis

Mutagenesis into the S-hydroxynitrile lyase (wild-SHNL) gene derived from cassava *(Manihot esculenta*) (SEQ ID NO: 1: Japanese Patent Application No. 2002-365675) was carried out using the GeneMorph^{®} PCR Mutagenesis Kit (Stratagene).
PCR was carried out using 600 ng of the pKK223-3/SHNL-Wild plasmid having the wild-SHNL gene incorporated into the multi-cloning site of pKK223-3 (Amersham Biosciences) as a template and the following oligo DNA as primers.
Forward primer: 5'-GGG GAA TTC ATG GTT ACT GCA CAC TTC GTT CTG ATT CAC-3' (SEQ ID NO: 11)
Reverse primer: 5'-GGG AAG CTT TTA AGC GTA TGC ATC AGC AAC TTC TTG CAG-3' (SEQ ID NO: 12)

### 2. Transformation

The resulting PCR product (SHNL-Mutants) was digested with *Eco*RI and *Hind*III restriction enzymes (Toyobo Co., Ltd.), and the resultant was ligated to the pKK223-3 vector, the multi-cloning site of which had also been digested with the *Eco*RI and *Hind*III restriction enzymes. The LigaFast^{™} Rapid DNA Ligation System (Promega) was used for ligation. The ligation reaction solution was applied to DH5α competent cells (Toyobo Co., Ltd.) for transformation, and a plurality of DH5α/pKK223-3/SHNL-Actmt strains were obtained.

### 3. Selection and recombination into high-expression vector

A plurality of DH5α/pKK223-3/SHNL-Actmt strains were cultured in test tubes, 1 ml each of the culture solution was fractionated, the fractionated culture solution was centrifuged to remove the supernatant, and cell pellets were obtained. The obtained cells were resuspended in 200 µl of sodium citrate buffer (pH 5.5) and then homogenized via an ultrasonic cell homogenizer. The homogenized cells were centrifuged at 15,000 rpm for 5 minutes to obtain a solution of homogenized cells. This solution of homogenized cells was heated at 60°C for 2 hours, and SHNL activity of the solution was then assayed. The SHNL activity was determined based on the amount of aldehyde generated per unit time upon mandelonitrile degradation by SHNL at 20°C. The amount of aldehyde generated per unit time is determined by measuring the increase in absorbance at 249.6 nm (with the use of a spectrophotometer, manufactured by Shimadzu Co., Ltd.).

On the basis of the results, the DH5α/pKK223-3/SHNL-Actmt001f2 strain that was still active after heating was selected as a thermostable strain. The selected strain was subjected to colony PCR, and the resulting PCR product was used as a template for sequencing. Based on the results of analyzing the reaction product, DHSa/pKK223-3/SHNL-Actmt001f2 was found to have a nucleotide derived from the nucleotide sequence as shown in SEQ ID NO: 1 by substitution of guanine by adenine at position 494. Thus, Actmt001f2-SHNL was found to be mutant SHNL having an amino acid sequence derived from the amino acid sequence of wild-SHNL by substitution of glycine by aspartic acid at position 165. Hereafter, this mutant SHNL (SHNL in which glycine at position 165 has been substituted with aspartic acid) is referred to as "Actmt-001f2-SHNL."

Subsequently, the SHNL-Actmt001f2 gene was introduced into the pET21 vector (Novagen) that permits high-level expression of proteins. The pKK223-3/SHNL-Actmt001f2 plasmid was prepared, and PCR was carried out using the resulting plasmid as a template, the primers shown below, and the DNA polymerase (KODplus, TOYOBO, Co., Ltd.) to remove the *Eco*RI and *Hind*III restriction enzyme sites that had been added to both terminuses of the template. The *Nde*I and *Bam*HI restriction enzyme sites were added instead.
Forward primer: 5'-GGG GGG GGG CAT ATG GTT ACT GCA CAC TTC GTT CTG ATT CAC AC-3' (SEQ ID NO: 13)
Reverse primer: 5'-GGG GGA TCC TTA AGC GTA TGC ATC AGC AAC TTC TTG CAG-3' (SEQ ID NO: 14)

The resulting PCR product was digested with *Nde*I (New England Bio Labs) and *Bam*HI restriction enzymes (Toyobo Co., Ltd.), and the resultant was ligated to the pET21 a vector (Novagen), the multi-cloning site of which had also been digested with the *Nde*I and *Bam*HI restriction enzymes. The LigaFast^{™} Rapid DNA Ligation System (Promega) was used for ligation. The ligation reaction solution was applied to BL21 (DE3) competent cells (Novagen) for transformation, and BL21(DE3)/pET21a/SHNL-Actmt001f2, which is the expression system for SHNL in which amino acid 165 has been substituted with Asp, was obtained.

### Reference Example 2: Experiment for thermostability of Actmt001f2-SHNL

### 1. Method of experimentation

### 1) Preparation of enzyme solution

*E. coli* strains, BL21(DE3)/pET21a/SHNL-Wild and BL21(DE3)/pET21a/SHNL-Actmt001f2, were cultured in 5 ml of LB medium at 37°C for 12 hours. The resulting culture broth (100 µl) was inoculated to 5 ml of NS-2 medium described below, and IPTG was added thereto to perform culture at 20°C for 60 hours. After the completion of culture, the culture solution was centrifuged to obtain cell pellets. The cell pellets were suspended in a 0.2 M sodium citrate buffer (pH 5.5) and cells were ultrasonically homogenized. The solution of homogenized cells was centrifuged, and the supernatant was recovered as enzyme solutions of wild-SHNL and Actmt-001f2-SHNL. The enzyme solution of wild-SHNL had an activity level of 74 U/ml and a protein concentration of 6.29 mg/ml. The enzyme solution of Actmt-001f2-SHNL had an activity level of 69 U/ml and a protein concentration of 5.96 mg/ml.

**Table 3**

| Composition of NS-2 medium (pH 6) | |
|---|---|
| Glycerol | 40 g |
| (NN₄)₂SO₄ | 10 g |
| KH₂PO₄ | 2 g |
| K₂HPO₄ | 6 g |
| Yeast Ext | 40 g |
| MgSO₄ | 1 g |
| Adekanol | 20 drops |
| Total | 1 liter (the total amount was adjusted to 1 liter with distilled water) |

The aforementioned medium was autoclaved, and filter-sterilized ampicillin (100 mg/l: final concentration) and filter-sterilized IPTG (238 mg/l: final concentration) was added.

### 2) Heat treatment of enzyme solution

The enzyme solutions of wild-SHNL and Actmt-001f2-SHNL (200 µl each) were placed in Eppendorf tubes and the enzyme solutions were heated to 45°C to 70°C in a heat block. After heat treatment for 30 minutes, the samples were recovered by centrifugation, and the level of residual activity was assayed in relation to the enzyme activity at the initiation of the reactions. The enzyme activity was assayed in the manner described in Reference Example 1.

### 2. Result of experiment

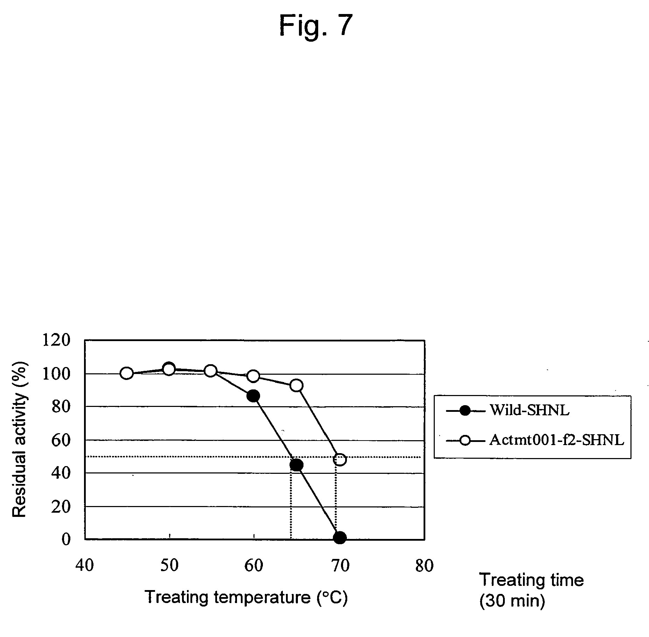

The activity of the solution of wild-SHNL was reduced to half the original level at 65°C; however, 90% or more activity of Actmt-001f2-SHNL remained (Fig. 7). The activity of Actmt-001f2-SHNL was reduced to half the original level at around 70°C. This indicated that heat resistance thereof was improved by approximately 5°C from that of wild-SHNL. Accordingly, heat resistance of *Manihot esculenta-*derived SHNL was found to improve by substitution of amino acid 165 from glycine to aspartic acid.

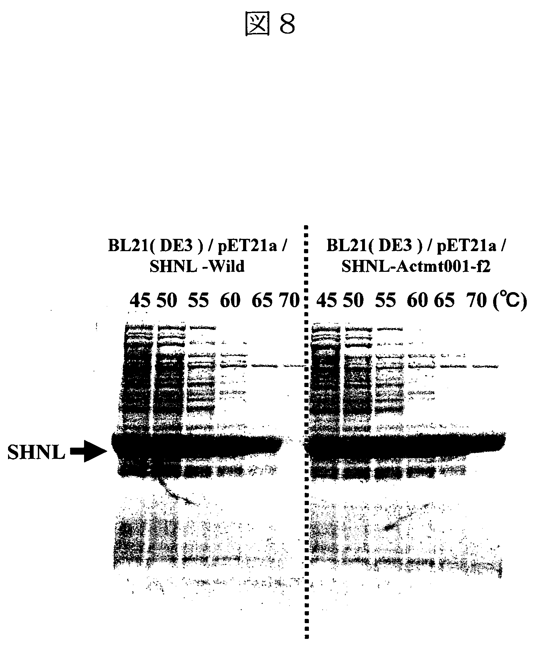

The heated enzyme solution sample was analyzed via SDS-PAGE (Fig. 8). Because the sample was heated and then centrifuged, denatured water insolubilized proteins were removed.

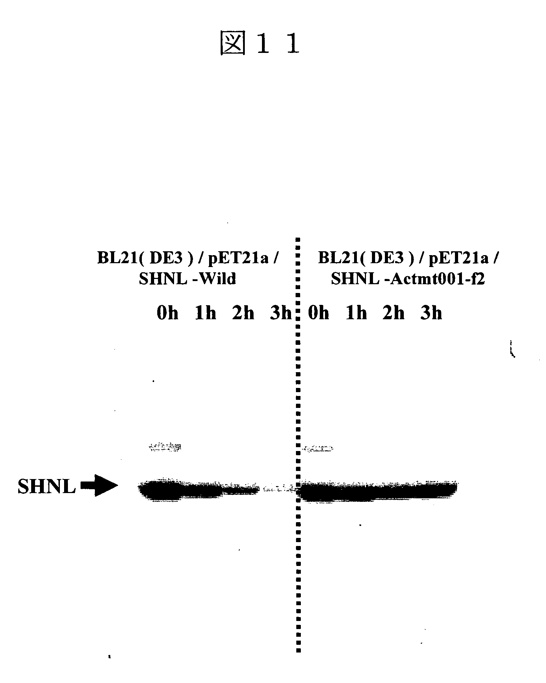

As shown in Fig. 5, the amount of enzyme in wild-SHNL begins to rapidly decrease at 60°C (see the band indicated by an arrow in Fig. 11), and it substantially disappears at 70°C. In contrast, the amount of enzyme in Actmt-001 f2-SHNL decreases. However, a sufficient amount of enzyme remains at 70°C. The results of SDS-PAGE are consistent with the results of assaying enzyme activity (Fig. 7).

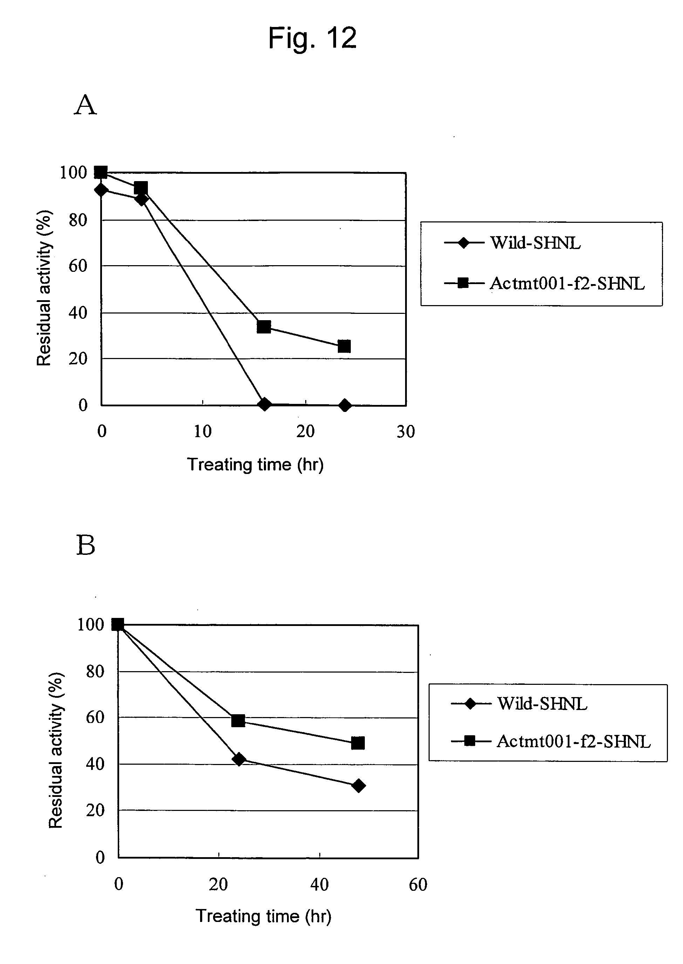

Fig. 6 shows the heat-induced changes in the protein concentration in the sample. The sample at 45°C contains a large quantity of proteins derived from an E. *coli* host (Figs. 11 and 12). Since such proteins are denatured and insolubilized in water via heating, they are removed from the sample as the temperature rises. Thus, the protein concentration in the wild-SHNL sample and that in the Actmt-001f2-SHNL decreased in an almost linear fashion as the temperature increased.

Generally, procedures such as gel filtration chromatography are required for enzyme purification. If Actmt-001f2-SHNL is heated, *E.* coli-derived proteins can be removed while maintaining enzyme activity via centrifugation or other means. Accordingly, it was considered that Actmt-001f2-SHNL could be simply and cost-effectively purified.

### Reference Example 3: Examination of changes in stability and protein concentration of Actmt-001f2-SHNL via heating at 60°C

In order to verify that E. coli-derived proteins can be actually removed while maintaining enzyme activity via heating, the following experiment was carried out.

### 1. Method of experimentation

### 1) Preparation of enzyme solution

*E. coli* strains, BL21(DE3)/pET2la/SHNL-Wild and BL21(DE3)/pET21a/SHNL-Actmt001f2, were cultured in 5 ml of LB medium at 37°C for 12 hours. The resulting culture solution (100 µl) was inoculated to 5 ml of NS-2 medium, and IPTG was added to perform culture at 20°C for 60 hours. After the completion of culture, the culture solution was centrifuged to recover cells. The recovered cells were suspended in a 0.2 M sodium citrate buffer (pH 5.5) and cells were ultrasonically homogenized. The solution of homogenized cells was centrifuged, the supernatant was recovered, and the enzyme solutions of wild-SHNL and Actmt-001f2-SHNL were obtained. The enzyme solution of wild-SHNL had an activity level of 83 U/ml and a protein concentration of 7.01 mg/ml. The enzyme solution of Actmt-001f2-SHNL had an activity level of 81 U/ml and a protein concentration of 6.65 mg/ml.

### 2) Heat treatment of enzyme solution

The enzyme solutions of wild-SHNL and Actmt-001f2-SHNL (200 µl each) were placed in Eppendorf tubes and the enzyme solutions were heated to 60°C in a heat block. The solutions were centrifuged every 30 minutes, 10 µl of each of the samples was recovered, and the level of residual activity and the protein concentration were assayed.

### 2. Result of experiment

### 1) Residual activity

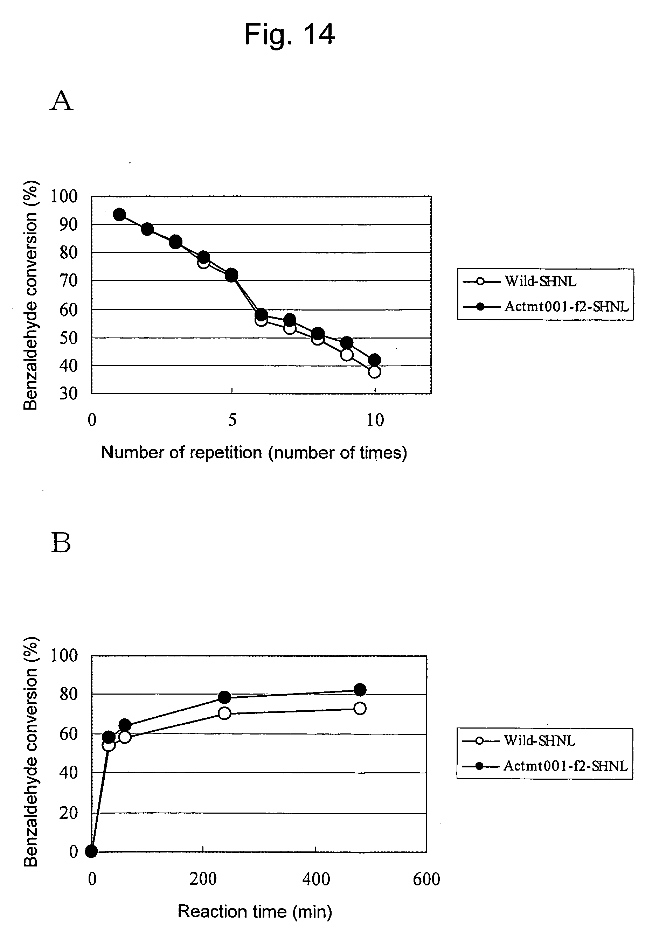

The activity of wild-SHNL was reduced to half the original level via heating for 1.5 hours; however, 75% of activity of Actmt-001f2-SHNL still remained after heating for 1.5 hours (Fig. 14).

### 2) Changes in protein concentration

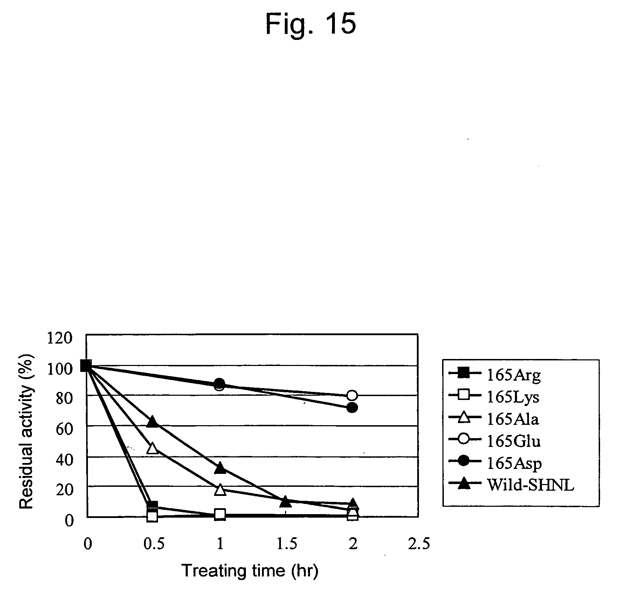

The resulting samples were analyzed via SDS-PAGE. Based on the results of SDS-PAGE (Fig. 15), the samples contained a large quantity of E. coli-derived proteins at 0 hours (without heating). In the case of the heated samples, however, *E.* coli-derived proteins were found to be removed from the wild-SHNL and Actmt-001f2-SHNL samples.

The protein concentration in the Actmt-001f2-SHNL sample after heating for 1 hour was 4.25 mg/ml, which was reduced to 64% of the initial level (Table 4). In contrast, the residual activity of Actmt-001f2-SHNL after heating for 1 hour was 80% or higher. Accordingly, it was found that E. coli-derived proteins could be removed from Actmt-001f2-SHNL while maintaining enzyme activity via heating. In the case of wild-SHNL, the protein concentration 1 hour later was 4.21 mg/ml, and the residual activity was 60%, which was the value reduced to 63% of the initial level.

**Table 4**

| Changes in stability and protein concentration (mg/ml-sample) via heating at 60°C | | | | |
|---|---|---|---|---|
| Heating time | Wild-SHNL | | Actmt-001 f2-SHNL | |
| 0 hr | 7.01 | (100) | 6.65 | (100) |
| 1 hr | 4.21 | (61) | 4.25 | (83) |
| 2 hr | 3.37 | (35) | 4.14 | (73) |

| | | | | |
|---|---|---|---|---|
| * Values in the parentheses represent the residual activity (%) at each time point | | | | |

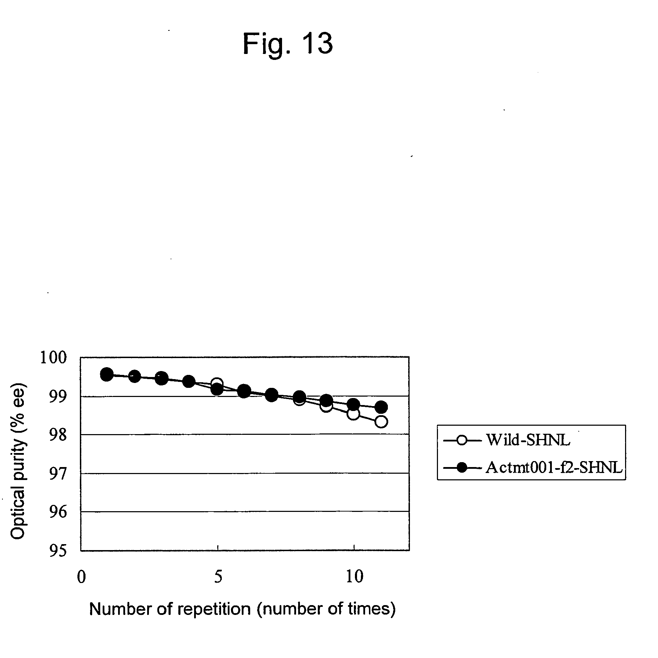

As is apparent from the data, wild-SHNL is disadvantageously denatured and inactivated with other contaminating proteins via heating at 60°C. Thus, separation and purification via heating at temperatures higher than this level is difficult. Although heating at 45°C to 55°C is feasible, the rate of contaminating proteins being denatured is very mild in such a temperature range, as is apparent from Fig. 13. Accordingly, a considerable amount of time would be required for sufficient separation and purification.

### Reference Example 4: Organic solvent resistance of Actmt-001f2-SHNL

In general, thermostability of enzymes is deeply involved with other environmental stresses such as stability in organic solvents. Thus, Actmt-001f2-SHNL can also have improved stability in organic solvents. Therefore, organic solvent resistance of Actmt-001f2-SHNL was examined.

### 1. Method of experimentation

### 1) Preparation of enzyme solution

An enzyme solution was prepared in the same manner as in Reference Example 2. When stress resistance of enzymes is assayed, contaminating proteins in the sample sometimes function as protecting agents, and resistance may appear to be improved. Thus, the aforementioned samples were diluted with bovine serum albumin and a buffer, all the samples were adjusted to have an activity level of 44.19 U/ml and a specific activity level of 6.50 U/mg, and influences of contaminating proteins were eliminated from the experimentation system.

### 2) Organic solvent treatment

Ethanol and ethyl acetate were added to the enzyme solution as organic solvents. The final concentration of ethanol was 30%, and that of ethyl acetate was 40%. Thereafter, the samples were stored for 50 hours with agitation. Centrifugation was carried out every several hours, 10 µl each of the supernatant (aqueous layer) was fractionated to form the samples, and the activity level was assayed.

### 2. Result of experimentation

Ethanol resistance (Fig. 12A) and ethyl acetate resistance (Fig. 12B) of Actmt-001f2-SHNL were superior to those of wild-SHNL.

### Reference Example 5: Production of optically active cyanohydrin by Actmt-001f2-SHNL

SHNL is an enzyme that catalyzes the reaction between an aldehyde or ketone and hydrocyanic acid to synthesize optically active cyanohydrin. The ability of Actmt-001f2-SHNL to catalyze this reaction was examined by comparison with that of wild-SHNL.

### 1. Method of experimentation

### 1) Preparation of enzyme solution

BL21(DE3)/pET21a/SHNL-Wild and BL21(DE3)/pET21a/SHNL-Actmt001f2 were cultured, the culture solutions were centrifuged to remove the supernatants, and cell pellets were obtained. A sodium citrate buffer (0.66 g, pH 5.5) was added to the cell pellets (0.33 g) for resuspension, and cells were homogenized via an ultrasonic cell homogenizer. The homogenized cells were centrifuged at 15,000 rpm for 5 minutes to obtain solutions of homogenized cells. The solutions of homogenized cells were heated at 50°C for 3 hours and then centrifuged. The supernatants were filtered through a 0.45-µm filter, followed by concentration via ultrafiltration. A sodium citrate buffer (pH 5.5) was added to each of these enzyme concentrates to adjust the activity levels as shown below. The prepared enzyme solution (0.3 ml) was mixed with 300 mg of silica gel to obtain immobilized enzymes.

**Table 5**

| Enzyme | Activity level (U/ml) | Specific activity level (U/mg) |
|---|---|---|
| Wild-SHNL | 250 | 31.02 |
| Actmt-001f2-SHNL | 250 | 27.16 |

### 2) Enzyme reaction

A 0.2 M citrate buffer (0.337 ml, pH 5.5) was added to 4.492 ml of t-butylmethylether and 1.61 M HCN. This solution was agitated for 30 minutes and then allowed to stand, followed by removal of an aqueous layer. This solution was added to a 9 ml screw vial containing 300 mg of the immobilized enzymes prepared above. Benzaldehyde (0.508 ml) was added, and the mixture was agitated via a bottle roller to perform an enzyme reaction. The reaction solution (4 ml) was recovered 1 hour after the initiation of the reaction. Subsequently, the same amount of HCN/t-butylmethylether solution, which had been subjected to the same treatment, was added, and the same amount of benzaldehyde was added to perform an enzyme reaction. The reaction solution (5 ml) was recovered 1 hour after the initiation of the reaction. This reaction was repeated and a total of 11 enzyme reactions were carried out. At the 11th reaction, the reaction time was extended to assay the process of enzyme reaction, and the process was analyzed.

### 2. Result of experimentation

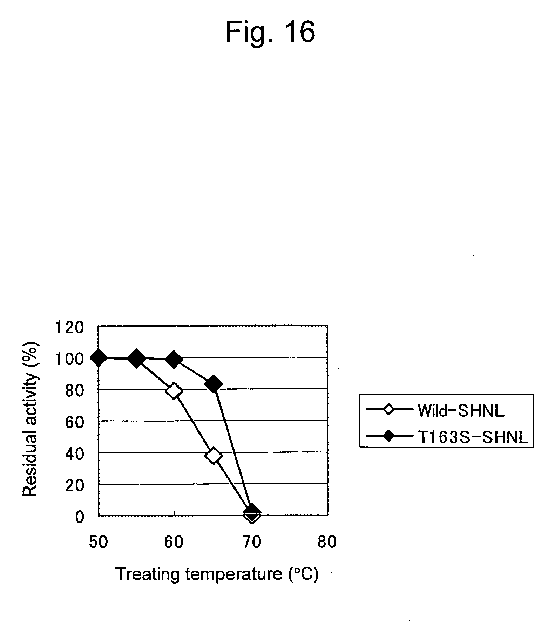

The thermostable enzyme (Actmt-001f2-SHNL) generated S-mandelonitrile at the same reaction rate as wild-SHNL. Thus, Actmt-001f2-SHNL was found to have capacity for synthesizing optically active cyanohydrin equivalent to that of wild-SHNL. As the reaction was repeatedly carried out, the reaction rates thereof gradually declined. The degree of the reaction rate decline was milder in the case of Actmt-001f2-SHNL (Fig. 16A).

As a result of comparing the processes of the 11th reaction, the reaction rate of Actmt-001f2-SHNL was improved by approximately 10% (Fig. 16B). This may result from a possibility such that stability of the thermostable enzyme (Actmt-001f2-SHNL) in the enzyme reaction system was improved in addition to the heat resistance.

### 3. Conclusion

Actmt-001f2-SHNL was found to be capable of synthesizing optically active cyanohydrin with productivity and optical purity that were the same as those of wild-SHNL. Further, approximately 10% of lifespan prolongation was observed via repeated reactions.

### Reference Example 6: Preparation of BL21(DE3)/pET21a/SHNL-G165E

In the thermostable enzyme Actmt-001f2-SHNL, position 165 of its amino acid sequence was substituted with an acidic amino acid (aspartic acid). The BL21(DE3)/pET21a/SHNL-G165E expression system for SHNL in which amino acid 165 had been substituted with an acidic amino acid (glutamic acid) was prepared.

### 1. Mutagenesis

In the same manner as in Reference Example 1, the QuikChange XL Site-Directed Mutagenesis Kit (Stratagene) was used for modifying amino acid 165. The pET21a/SHNL-Wild plasmid (10 ng) was used as a template, and the following oligo DNAs were used as primers to perform elongation. The resulting reaction product was digested with the *Dpn*I restriction enzyme included in the kit.
Forward primer: 5'-CGT GAA AAC CTG TTC ACC AAA TGC ACT GAT GAA GAA TAT GAA CTG GCA AAA ATG-3' (SEQ ID NO: 15)
Reverse primer: 5'-CAT TTT TGC CAG TTC ATA TTC TTC ATC AGT GCA TTT GGT GAA CAG GTT TTC ACG-3' (SEQ ID NO: 16)

### 2. Transformation

The resulting reaction product processed with restriction enzymes was applied to XL10-Gold competent cells included in the kit for transformation, and the obtained cells were subjected to colony PCR. The resulting PCR product was used as a template for sequencing, and the reaction product was analyzed. Based on the analysis, a cell in which GC at positions 494 and 495 in the nucleotide sequence had been mutated by AA was selected. The pET21a/SHNL-G165E plasmid was prepared therefrom, applied to BL21 (DE3) competent cells (Novagen) for transformation, and the BL21(DE3)/pET21a/SHNL-G165E expression system for SHNL, in which amino acid 165 had been substituted with Glu, was prepared.

### Reference Example 7: Changes in heat resistance depending on amino acid type at substitution site

Position 165 in the amino acid sequence of SHNL was substituted with various types of polar amino acids, and subsequent effect on heat resistance of SHNL was examined.

### 1. Method of experimentation

In accordance with Reference Example 1 and Reference Example 6, mutagenesis into 165-Gly was carried out using the QuikChange XL Site-Directed Mutagenesis Kit (Stratagene), and the following mutants were prepared.
i) DH5α/pKK223-3/Actmt001f2-Glu (substitution of amino acid 165 with glutamic acid)
ii) DH5α/pKK223-3/Actmt001f2-Lys (substitution of amino acid 165 with lysine)
iii) DH5α/pKK223-3/Actmt001f2-Arg (substitution of amino acid 165 with arginine)
iv) DH5α/pKK223-3/Actmt001f2-Ala (substitution of amino acid 165 with alanine)

Glutamic acid is an amino acid that has an acidic residue, as does aspartic acid. Lysine and arginine are basic amino acids. Alanine is a neutral amino acid, as is glycine. These four mutants, DH5α/pKK223-3/SHNL-Actmt001-f2, and SHNL-Wild (6 strains in total) were subjected to the heating test in the same manner as in Reference Example 2.

### 2. Results of experimentation

As a result of the heating test in accordance with Reference Example 2, the mutant SHNL exhibited roughly 3 types of heat resistances, depending on different properties of amino acid residues that had been introduced (Fig. 15).

### 1) Substitution with basic amino acid (Arg, Lys)

Activity of the mutant SHNL substantially completely disappeared in 30 minutes. Heat resistance was apparently deteriorated, compared with that of wild-SHNL.

### 2) Substitution with neutral amino acid (Ala)

Mutant SHNL exhibited heat resistance equivalent to that of wild-SHNL (165-Gly, neutral).

### 3) Substitution with acidic amino acid (Glu)

The activity level changed in the substantially same manner as with the case of Actmt-001f2-SHNL (165-Asp, acidic). The highest level of heat resistance was exhibited among three types of amino acids.

As is apparent from the above results, heat resistance of mutant SHNL in which amino acid 165 had been substituted with an acidic amino acid improved. In contrast, heat resistance of mutant SHNL in which amino acid 165 had been substituted with a basic amino acid significantly deteriorated.

### Reference Example 8: Modification of helix D3':

### Preparation of BL21 (DE3)/pET21a/SHNL-SD 173-1 e9

Amino acids 165 to 173 of helix D3' (163-174) are positioned crosswise to amino acids 17 to 21 of helix A, and they are adjacent to each other. Amino acid substitution in such regions can influence the level of heat resistance. Thus, Val as amino acid 173 of the amino acid sequence of SHNL was substituted with Leu, and the resulting influences on thermostability of SHNL were examined.

### 1. Mutagenesis

In accordance with Reference Example 1 and Reference Example 6, SHNL (SEQ ID NO: 16), in which amino acid 173 had been substituted with Leu, was prepared using the QuikChange XL Site-Directed Mutagenesis Kit (Stratagene).

Elongation reaction was carried out using the pET21a-SHNL-Wild plasmid (10 ng) as a template and the following oligo DNA as primers. Subsequently, the resulting reaction product was digested with the *Dpn*I restriction enzyme included in the kit.
Forward primer: 5'-GGC GAA TAT GAA CTG GCA AAA ATG NNN ATG CGC AAG GGC TCT CTG-3' (SEQ ID NO: 17)
Reverse primer: 5'-CAG AGA GCC CTT GCG CAT NNN CAT TTT TGC CAG TTC ATA TTC GCC-3' (SEQ ID NO: 18)

### 2. Transformation and heat resistance assay

The resulting reaction product processed with restriction enzymes was applied to XL10-Gold competent cells included in the kit for transformation, and all the obtained colonies formed on an LB (Amp) plate were suspended in LB (Amp) liquid medium. The pET21a/SHNL-SD173-1NNNMutant plasmids were prepared from the resulting suspension and then added to BL21 (DE3) competent cells (Novagen) for transformation to prepare BL21(DE3)/pET21a/SHNL-SD173-1NNNMutant strains.

Multiple BL21(DE3)/pET21a/SHNL-SD173-1NNNMutant strains were cultured in test tubes, 1 ml each of the culture broth was fractionated, the fractionated culture broth was centrifuged to remove the supernatant, and cell pellets were obtained. The obtained cells were resuspended in 200 µl of sodium citrate buffer (pH 5.5) and then homogenized via an ultrasonic cell homogenizer. The homogenized cells were centrifuged at 15,000 rpm for 5 minutes to obtain a solution of homogenized cells. This solution of homogenized cells was heated at 60°C for 2 hours, and SHNL activity of the solution was then assayed. On the basis of the assay result, BL21(DE3)/pET21a/SHNL-SD173-1e9 and other 3 strains that were still active after heating were selected as thermostable strains. The selected strains were subjected to colony PCR, and the resulting PCR product was used as a template for sequencing. Based on the results of analyzing the reaction product, SHNL-SD173-1e9 was found to have a nucleotide sequence (SEQ ID NO: 15) in which amino acids 517 to 519, i.e., GTT(V), of the nucleotide sequence had been mutated to CTG(L) and valine as amino acid 173 had been substituted with leucine. Hereafter, SHNL-SD173-1e9 is referred to as SHNL-V173L. All of the other three strains were found to be mutants in which valine as amino acid 173 had been substituted with leucine.

### Reference Example 9: Evaluation of heat resistance of V173L-SHNL

Thermostability of V173-SHNL was compared with that of wild-SHNL and that of Actmt001-f2-SHNL.

### 1. Method of experimentation

### 1) Preparation of enzyme solution

*E. coli* strains, i.e., BL21(DE3)/pET21a/SHNL-Wild, BL21(DE3)/pET21a/SHNL-Actmt001-f2, and BL21(DE3)/pET21a/SHNL-V173L, were cultured in the same manner as in Reference Example 2 to obtain enzyme solutions. The aforementioned samples were diluted with bovine serum albumin and a buffer, all the samples were adjusted to have an activity level of 17.6 U/ml, a specific activity level of 4.5 U/mg, and a protein concentration of 3.9 mg/ml, and influences of contaminating proteins were eliminated from the experimentation system.

### 2) Heat treatment of enzyme solution

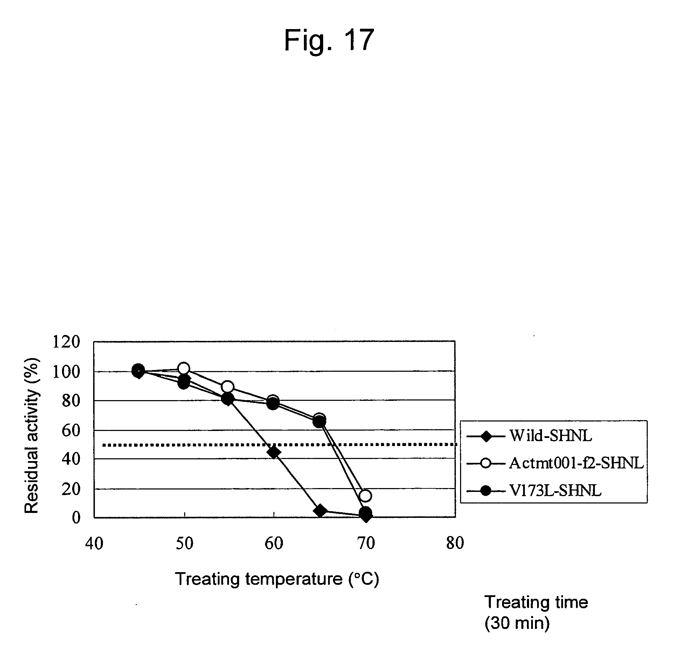

The enzyme solutions of wild-SHNL, Actmt001-f2-SHNL, and V173L-SHNL (200 µl each) were placed in Eppendorf tubes and the enzyme solutions were heated to 45°C to 70°C in a heat block. After heat treatment for 30 minutes, the samples were recovered by centrifugation, and the level of residual activity was assayed (Fig. 17).

As a result, the temperature at which enzyme activity of the sample was reduced to half the original level was 60°C in the case of wild-SHNL, and that of V173L-SHNL and that of Actmt001-f2-SHNL were around 65°C, which indicates that these samples had improved heat resistance by approximately 5°C from wild-SHNL. Thus, V173L-SHNL was found to have heat resistance that was equivalent to that of Actmt001-f2-SHNL.

Amino acid 173 of SHNL, i.e., valine, is adjacent to the amino acid of another monomer, i.e., valine, at the time of dimer formation (the distance between terminuses is approximately 4.5 angstroms). Substitution of valine with leucine results in extension of amino acid residue 173 by one carbon. Accordingly, the distance between residues can narrow when carbon chains elongate, and hydrophobic interaction between non-polar amino acid residues can be reinforced.

### Reference Example 10: Organic solvent resistance of mutant enzyme V 173L-SHNL

As mentioned in Reference Example 4, ethanol resistance and ethyl acetate resistance of the thermostable mutant enzyme (Actmt001-f2-SHNL) were superior to those of wild-SHNL.

The mutant enzyme (V173L-SHNL) mentioned in Reference Example 9 has thermostability that is substantially the same as that of Actmt001-f2-SHNL. Thus, the resistance of V173L-SHNL to ethanol and to ethyl acetate was also examined.

### 1. Method of experimentation

### 1) Preparation of enzyme solution

*E. coli* strains, i.e., BL21(DE3)/pET21a/SHNL-Wild, BL21(DE3)/pET21a/SHNL-Actmt001-f2, and BL21(DE3)/pET21a/SHNL-V173L, were cultured in the same manner as in Reference Example 2 to prepare enzyme solutions. The prepared enzyme solutions were diluted with bovine serum albumin and a 0.2 M sodium citrate buffer, all the samples were adjusted to have an activity level of 45 U/ml and a specific activity level of 6.5 U/mg, and influences of contaminating proteins were eliminated from the experimentation system.

### 2) Organic solvent treatment

The enzyme solutions were treated with ethanol and with ethyl acetate in the same manner as in Reference Example 4, and the residual activity was assayed.

### 2. Result of Experimentation

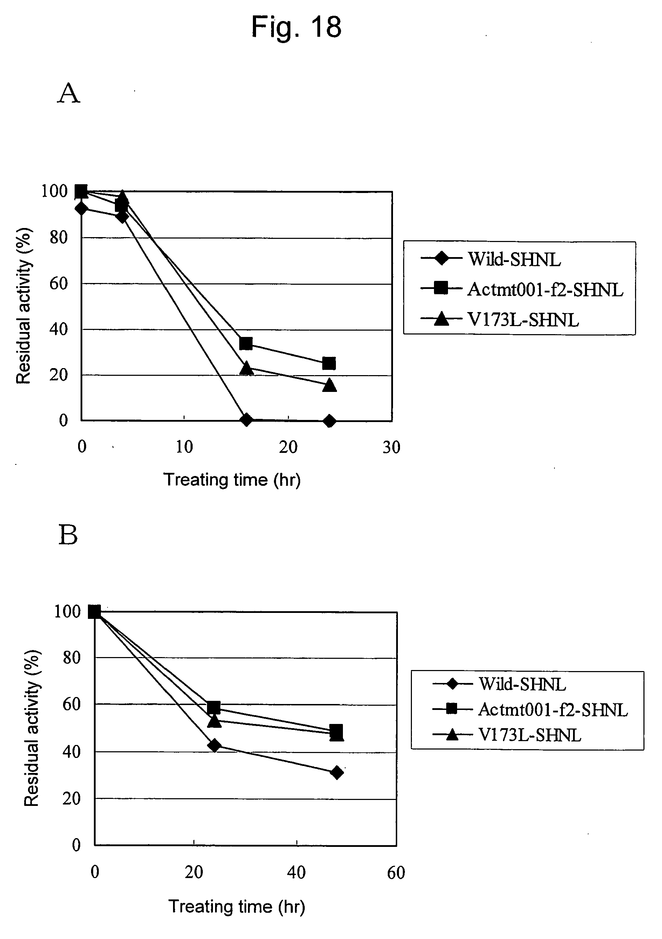

V173L-SHNL was found to be more resistant to ethanol (Fig. 18A) and to ethyl acetate (Fig. 18B) than wild-SHNL. Further, V173L-SHNL was more resistant to ethanol than Actmt001-f2-SHNL. While the residual activity after 16 hours of Actmt001-f2-SHNL treatment was 23%, V173L-SHNL was 34%. Resistances of two types of mutant enzymes to ethyl acetate were at substantially the same level.

Reference Example 11: Acquisition of mutant enzyme Actmt020-b8-SHNL

### 1. Mutagenesis

In the same manner as in Reference Example 1, mutagenesis into the wild-SHNL gene was carried out using the GeneMorph^{™} PCR Mutagenesis Kit. The template and the primers employed in Reference Example 1 were employed herein.

### 2. Transformation

In the same manner as in Reference Example 1, the resulting PCR product was ligated to the pKK223-3 vector and applied to DH5α competent cells for transformation to obtain a plurality of DH5α/pKK223-3/SHNL-Actmt020 strains.

### 3. Selection of thermostable enzyme and sequence analysis

In the same manner as in Reference Example 1, the DH5α/pKK223-3/SHNL-Actmt020-b8 strain that had been still active after heating was selected. Colony PCR was carried out using primers as shown in SEQ ID NO: 11 and SEQ ID NO: 12 and the selected strain was used as a template. Further, sequencing was carried out using the resulting PCR product as a template and the same primers. The analysis of the reaction product demonstrated that SHNL-Actmt020-b8 had a nucleotide sequence derived from the nucleotide sequence as shown in SEQ ID NO: 1 by modification of amino acid 520 from adenine to thymine. Accordingly, it was verified that SHNL-Actmt020-b8 was a mutant SHNL having an amino acid sequence derived from the amino acid sequence of wild-SHNL by substitution of methionine as amino acid 174 with leucine. Hereafter, this mutant SHNL is referred to as "Actmt020-b8-SHNL."

### Reference Example 12: Thermostability of mutant enzyme Actmt020-b8-SHNL

### 1. Method of experimentation

### 1) Preparation of enzyme solution

The *E. coli* strain, DH5α/pKK223-3/SHNL-Actmt020-b8, which had been constructed in Reference Example 11, and the DH5α/pKK223-3/SHNL-Wild strain as a control were both cultured in the same manner as in Reference Example 2 to prepare enzyme solutions. The prepared enzyme solutions were diluted with bovine serum albumin and a 0.2 M sodium citrate buffer, all the samples were adjusted to have an activity level of 3.15 U/ml and a protein concentration of 1.38 mg/ml, and influences of contaminating proteins were eliminated from the experimentation system.

### 2) Heat treatment of enzyme solution

The enzyme solutions were heated to 60°C in the same manner as in Reference Example 3. The solutions were centrifuged every 30 minutes after the initiation of heating, and the supernatant was subjected to assay of residual activity in relation to the enzyme activity before heating.

### 2. Result of experimentation

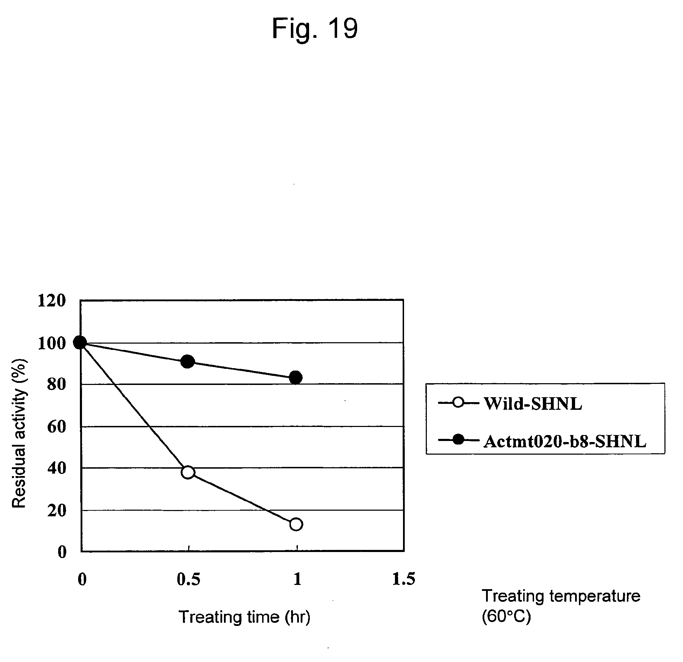

Actmt020-b08-SHNL exhibited significantly improved thermostability compared with that of wild-SHNL (Fig. 19). Thus, it was found that substitution of methionine as amino acid 174 that constitutes helix D3' with leucine could improve the thermostability of SHNL.

### Reference Example 13: Acquisition of mutant enzyme Actmt022-g12-SHNL

### 1. Mutagenesis

In the same manner as in Reference Example 1, mutagenesis into the wild-SHNL gene was carried out using the GeneMorph^{™} PCR Mutagenesis Kit. The template and the primers employed in Reference Example 1 were employed.

### 2. Transformation

In the same manner as in Reference Example 1, the resulting PCR product was ligated to the pKK223-3 vector and applied to DH5α competent cells for transformation to obtain a plurality of DH5α/pKK223-3/SHNL-Actmt022 strains.

### 3. Selection of thermostable enzyme and sequence analysis

In the same manner as in Reference Example 1, the DH5α/pKK223-3/SHNL-Actmt022-g12 strain that had been still active after heating was selected. Colony PCR was carried out using primers as shown in SEQ ID NO: 11 and SEQ ID NO: 12 and the selected strain was used as a template. Further, sequencing was carried out using the resulting PCR product as a template and the same primers. The analysis of the reaction product demonstrated that SHNL-Actmt022-g12 had a nucleotide sequence derived from the nucleotide sequence as shown in SEQ ID NO: 1 by modification of amino acid 63 from adenine to thymine. Accordingly, it was verified that Actmt022-g12-SHNL was a mutant SHNL having an amino acid sequence derived from the amino acid sequence of wild-SHNL by substitution of lysine as amino acid 21 with asparagine. Lysine at position 21 in the amino acid sequence was an amino acid that constitutes helix A where a dimer is formed.

### Reference Example 14: Construction of mutant enzyme with amino acid substitution at Lys-21

The position 21 in the amino acid sequence of SHNL was substituted with various types of amino acids, and influences thereof on heat resistance of SHNL were examined.

### 1) Mutagenesis

In the same manner as in Reference Example 8, the QuikChange XL Site-Directed Mutagenesis Kit (Stratagene) was employed. Extension reaction was carried out using 10 ng of pET21 a/SHNL-Wild as a template and primers as shown in SEQ ID NO: 19 and in SEQ ID NO: 20. Subsequently, the resulting reaction product was digested with the *Dpn*I restriction enzyme included in the kit.
ggcgcatgga tttggcacnn nctgaaaccg gccctggaa (SEQ ID NO: 19)
ttccagggcc ggtttcagnn ngtgccaaat ccatgcgcc (SEQ ID NO: 20)

### 2) Transformation

The resulting reaction product processed with restriction enzymes was applied to XL10-Gold competent cells included in the kit for transformation and then cultured on an LB (Amp) plate. The resulting colonies formed on the plate were resuspended in LB (Amp) liquid medium to prepare the pET21a/SHNL-SDLys21NNN plasmid. This plasmid was applied to BL21 (DE3) competent cells (Novagen) for transformation to prepare a plurality of BL21 (DE3)/pET21 a/SHNL-SDLys21NNN strains.

### 3) Selection of mutant SHNL

The prepared *E. coli* BL21(DE3)/pET21a/SHNL-SDLys21NNN strains were cultured in the same manner as in Reference Example 2. The resulting culture broths were employed to select mutants having improved thermostability in accordance with the method of Reference Example 1. As a result, three types of mutants, i.e., BL21(DE3)/pET21a/SHNL-SDLys21-RAM1, BL21(DE3)/pET21a/SHNL-SDLys21-RAM6, and BL21(DE3)/pET21a/SHNL-SDLys21-RAM8, were found to be active after heating. Subsequently, colony PCR was carried out using primers as shown in SEQ ID NO: 11 and in SEQ ID NO: 12 and the selected strains as templates. Further, sequencing was carried out using the resulting PCR product as a template and the same primers. The analysis of the reaction product demonstrated that SHNL-SDLys21-RAM1 had a nucleotide sequence (SEQ ID NO: 25) derived from the nucleotide sequence as shown in SEQ ID NO: 1 by substitution of adenine as amino acid 61 with guanine. Accordingly, it was verified that SDLys21-RAM1-SHNL was a mutant SHNL having an amino acid sequence derived from the amino acid sequence of wild-SHNL by substitution of lysine as amino acid 21 with glutamic acid. Similarly, SHNL-SDLys21-RAM6 had a nucleotide sequence derived from the nucleotide sequence as shown in SEQ ID NO: 1 by substitution of AAA as amino acids 61 to 63 with GAC. Accordingly, it was verified that SDLys21-RAM6-SHNL was a mutant SHNL having an amino acid sequence derived from the amino acid sequence of wild-SHNL by substitution of lysine as amino acid 21 with aspartic acid. Further, SHNL-SDLys21-RAM8 had a nucleotide sequence derived from the nucleotide sequence as shown in SEQ ID NO: 1 by substitution of adenine as amino acid 63 with cytosine. Accordingly, it was verified that SDLys21-RAM8 SHNL was a mutant SHNL having an amino acid sequence derived from the amino acid sequence of wild-SHNL by substitution of lysine as amino acid 21 with asparagine. Hereafter, SDLys21-RAM1 SHNL is referred to as "K21E-SHNL," RAM6 is referred to as "K21D-SHNL," and RAM8 is referred to as "K21N-SHNL."

### Reference Example 15: Heat resistance of mutant enzymes K21E-SHNL, K21D-SHNL, and K21N-SHNL

### 1. Method of experimentation

### 1) Preparation of enzyme solution

The E. *coli* strains, BL21(DE3)/pET21a/SHNL-K21E, BL21(DE3)/pET21a/SHNL-K21D, and BL21(DE3)/pET21a/SHNL-K21N,constructed in Reference Example 14 and the BL21(DE3)/pET21a/SHNL-Wild strain as a control were cultured in the same manner as in Reference Example 2 to prepare enzyme solutions. The prepared enzyme solutions were diluted with bovine serum albumin and a 0.2 M sodium citrate buffer, all the samples were adjusted to have an activity level of 11 U/ml and a protein concentration of 6.8 mg/ml, and influences of contaminating proteins were eliminated from the experimentation system.

### 2) Heat treatment of enzyme solution

The enzyme solutions were heated to 45°C to 65°C in the same manner as in Reference Example 2. After heat treatment for 30 minutes, the solutions were centrifuged, and the supernatant was subjected to assay of residual activity in relation to the enzyme activity before heating.

### 2. Result of experimentation

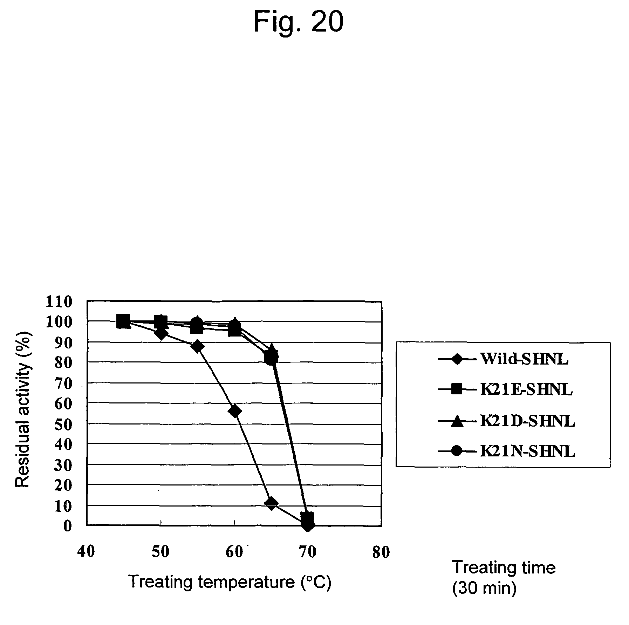

K21E-SHNL, K21D-SHNL, and K21N-SHNL exhibited significantly improved thermostability compared with that of wild-SHNL (Fig. 20). Thus, it was found that substitution of lysine as amino acid 21 that constitutes helix A with glutamic acid, aspartic acid, or asparagine could improve thermostability of SHNL.

### Reference Example 16: Preparation of SHNL genes, i.e., SHNL-G165E,V173L and SHNL-G165E,V173L,M174L, having multiple modification sites

Mutant SHNL, i.e., Actmt001-f2-SHNL, V173L-SHNL, and Actmt020-b8-SHNL, each independently possessed a single amino acid modification site. Further their heat and organic solvent resistance were superior to those of wild-SHNL. In order to further improve heat resistance and organic solvent resistance, modification sites of individual strains of these strains were introduced in combination in a single gene.

### 1. Construction of SHNL gene, i.e., SHNL-G165E,V173L, having multiple modification sites

### 1) Mutagenesis

In the same manner as in Reference Example 8, the QuikChange XL Site-Directed Mutagenesis Kit (Stratagene) was employed. Extension reaction was carried out using 10 ng of pET21a/SHNL-SD173-le9 plasmid as a template and primers as shown in SEQ ID NO: 15 and in SEQ ID NO: 16. Subsequently, the resulting reaction product was digested with the *Dpn*I restriction enzyme included in the kit.

### 2) Transformation

The resulting reaction product processed with restriction enzymes was applied to XL10-Gold competent cells included in the kit for transformation. The resulting colonies formed on the LB (Amp) plate were recovered and then cultured in LB (Amp) liquid medium at 37°C for 12 hours. Plasmids were isolated from the culture broth and an extension reaction was carried out using the resulting plasmids as a template and primers as shown in SEQ ID NO: 11 and in SEQ ID NO: 12. Further, sequencing was carried out using the resulting reaction product as a template and the same primers. According to the results of analyzing the reaction product, the pET21a/SHNL-G165E,V173L plasmid having the SHNL genes with 2 amino acid mutations (Gly-to-Glu at position 165 and Val-to-Leu at position 173) were selected. The selected plasmid was applied to BL21 (DE3) competent cells (Novagen) for transformation to prepare the BL21(DE3)/pET21a/SHNL-G165E,V173L strain.

### 2. Construction of SHNL gene, i.e., SHNL-G165E,V173L,M174L, having multiple modification sites

### 1) Mutagenesis

In the same manner as in Reference Example 8, the QuikChange XL Site-Directed Mutagenesis Kit (Stratagene) was employed. The extension reaction was carried out using 10 ng of pET21a/SHNL-G165E,V173L plasmid as a template and primers as shown in SEQ ID NO: 21 and in SEQ ID NO: 22. Subsequently, the resulting reaction product was digested with the *Dpn*I restriction enzyme included in the kit.
tatgaactgg caaaaatgct gctgcgcaag ggctctctgt tc (SEQ ID NO: 21)
gaacagagag cccttgcgca gcagcatttt tgccagttca ta (SEQ ID NO: 22)

### 2) Transformation

The resulting reaction product processed with restriction enzymes was applied to XL10-Gold competent cells included in the kit for transformation. The resulting colonies formed on the LB (Amp) plate were recovered and then cultured in LB (Amp) liquid medium at 37°C for 12 hours. Plasmids were isolated from the culture solution reaction was carried out using the resulting plasmids as a template and primers as shown in SEQ ID NO: 11 and in SEQ ID NO: 12. Further, sequencing was carried out using the resulting reaction product as a template and the same primers. According to the results of analysis of the reaction product, the pET21a/SHNL-G165E,V173L,M174L plasmid having the SHNL genes with 3 amino acid mutations (Gly-to-Glu at position 165, Val-to-Leu at position 173, and Met-to-Leu at position 174) was selected. The selected plasmid was applied to BL21 (DE3) competent cells (Novagen) for transformation to prepare the BL21 (DE3)/pET21a/SHNL-G165E,V173L,M174L strain.

### Reference Example 17: Thermostability of SHNL genes, i.e., G165E,V173L,M174L-SHNL and G165E,V173L,M174L-SHNL, having multiple mutation sites

### 1. Method of experimentation

### 1) Preparation of enzyme solution

The E. *coli* strains constructed in Reference Example 16, i.e., BL21 (DE3)/pET21a/SHNL-G165E, BL21 (DE3)/pET21a/SHNL-V 173 L, BL21 (DE3)/pET21a/SHNL-G 165E, BL21 (DE3)/pET21 a/SHNL-V173L, and BL21(DE3)/pET21a/SHNL-M174L, as well as BL21(DE3)/pET21a/SHNL-Wild, were cultured in the same manner as in Reference Example 2 to prepare enzyme solutions. The prepared enzyme solutions were diluted with bovine serum albumin and a 0.2 M sodium citrate buffer, all the samples were adjusted to have an activity level of 70 U/ml and a protein concentration of 6 mg/ml, and influences of contaminating proteins were eliminated from the experimentation system.

### 2) Heat treatment of enzyme solution

The enzyme solutions were heated to 45°C to 75°C in the same manner as in Reference Example 2. After heat treatment for 30 minutes, the enzyme solutions were centrifuged, and the supernatant was subjected to assay of residual activity in relation to the enzyme activity before heating.

### 2. Result of experimentation

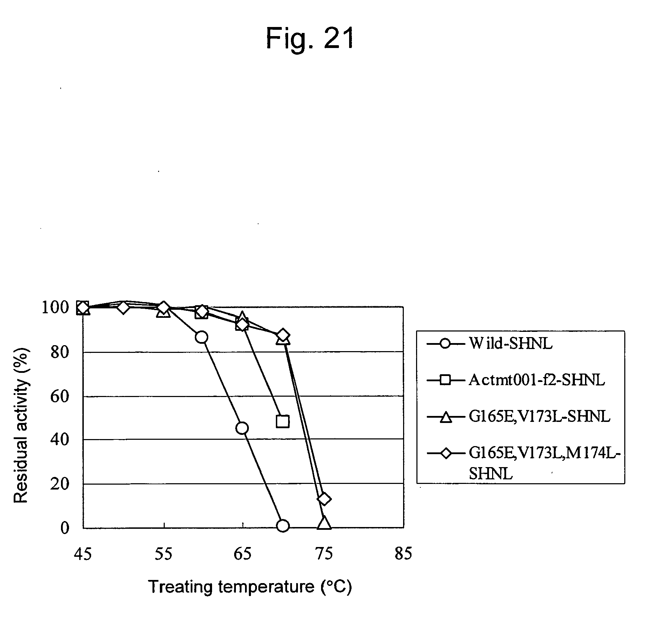

G165E,V173L-SHNL and G165E,V173L,M174L-SHNL exhibited significantly improved thermostability compared with that of wild-SHNL, and the level of residual activity at 70°C was approximately 90% in both cases (Fig. 21). In the case of G165E,V173L-SHNL, the activity level was rapidly inactivated at 75°C and the level of residual activity was 2%. G165E,V173L,M174L-SHNL with 3 modification sites retained 13% of its activity at 75°C. Thus, it was found that thermostability could be further improved by aggregating modification sites of individual strains on a single gene.

### Reference Example 18: Organic solvent resistance of SHNL genes, i.e., SHNL-G165E,V173L-SHNL and G165E,V173L,M174L-SHNL, having multiple substitution sites

### 1. Method of experimentation

### 1) Preparation of enzyme solution

The *E. coli* strains, i.e., BL21 (DE3)/pET21 a/SHNL-Wild, BL21(DE3)/pET21a/SHNL-G 165E,V173L, as well as BL21(DE3)/pET21a/SHNL-G165E,V173L,M174L, were cultured in the same manner as in Reference Example 2 to prepare enzyme solutions. The prepared enzyme solutions were diluted with bovine serum albumin and a 0.2 M sodium citrate buffer, all the samples were adjusted to have an activity level of 45 U/ml and a specific activity level of 6.5 mg/ml, and influences of contaminating proteins were eliminated from the experimentation system.

### 2) Organic solvent treatment

The enzyme solutions were treated with ethanol and with ethyl acetate in the same manner as in Reference Example 4, and the residual activity was assayed.

### 2. Result of Experimentation

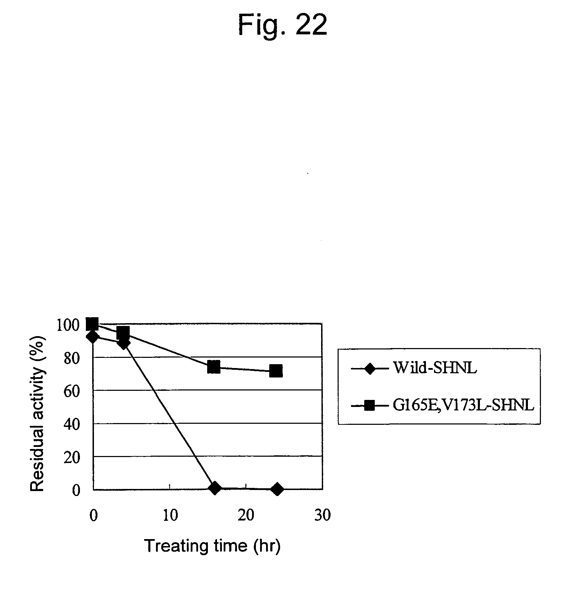

The enzyme solutions were treated with ethanol. As a result, activity of wild-SHNL substantially disappeared 16 hours after the initiation of treatment. However, G165E,V173-SHNL having multiple substitution sites retained as much as 73% of its activity (Fig. 22). As mentioned in Reference Example 4 and in Reference Example 11, Actmt001-f2,V173L-SHNL having a single amino acid mutation retained 20% to 30% of its resistance to ethanol after 16 hours of treatment. Thus, the ethanol resistance of SHNL having multiple substitution sites was found to be significantly improved.

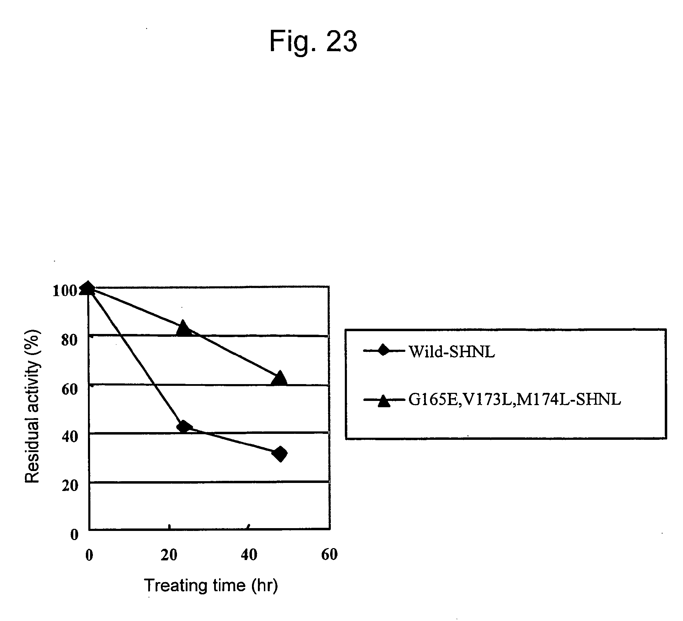

With the use of ethyl acetate, G165E,V173L,M174L-SHNL retained as much as 80% of its activity after 24 hours of treatment (Fig. 23). As with the case of ethanol resistance, organic solvent resistance was significantly improved via aggregation of modification sites of individual strains.

### Reference Example 19: Synthesis of optically active cyanohydrin using G165E,V173-SHNL enzyme having multiple modification sites

Optically active cyanohydrin was repeatedly synthesized using the multiple modification site bearing G165E,V173-SHNL enzyme. Stability in the enzyme reaction system was examined. Modification may alter substrate specificity or may deteriorate the capacity for asymmetric synthesis. Optically active cyanohydrin can be produced by common SHNL. Accordingly, this phenomenon was examined in the case at hand.

### 1. Method of experimentation

### 1) Preparation of enzyme solution

The *E. coli* strains, BL21 (DE3)/pET21a/SHNL-Wild and BL21(DE3)/pET21a/SHNL-G165E,V173L, were cultured in the same manner as in Reference Example 2 to prepare enzyme solutions. Further, a sodium citrate buffer (pH 5.5) was added to these enzyme solutions to adjust the activity levels to 500 U/ml. BSA was added to the G165E,V173-SHNL enzyme solution to bring the total protein concentration to the same level as that of wild-SHNL. Silica gel (300 mg) was mixed with 0.3 ml of such enzyme solution to obtain immobilized enzymes.

### 2) Enzyme reaction

Enzyme reaction was carried out under the reaction conditions described in Reference Example 5. 2-Chlorobenzaldehyde (2CBA) was used as a reaction substrate at a final concentration of 1.0 M instead of benzaldehyde. The samples were recovered every hour, and the 2CBA concentration of the reaction solution and the concentration of (R/S)-2-chloromandelonitrile were assayed. The time at which the conversion rate of 2-chlorobenzaldehyde exceeded 95% was defined as marking the completion of reaction, and 4 ml of the reaction solution was recovered after the completion of the reaction. Subsequently, the same amount of HCN/t-butylmethylether solution, which had been subjected to the same processing, was added, and the same amount of benzaldehyde was added to perform the second enzyme reaction. At the second and later reaction cycles, 5 ml of the reaction solution was recovered after the completion of the reaction. This enzyme reaction was repeated 4 times.

### 2. Result of experimentation

### 1) Optical purity

G165E,V173L-SHNL produced (S)-2-chloromandelonitrile with optical purity of 95% ee on average through 4 repeated reactions. Similarly, wild-SHNL exhibited optical purity of approximately 95% ee. Accordingly, G165E,V173L-SHNL was found to have the capacity for producing optically active cyanohydrin, which is substantially the same as that of wild-SHNL in terms of optical purity.

### 2) Comparison of reaction rate and degree of activity decrease

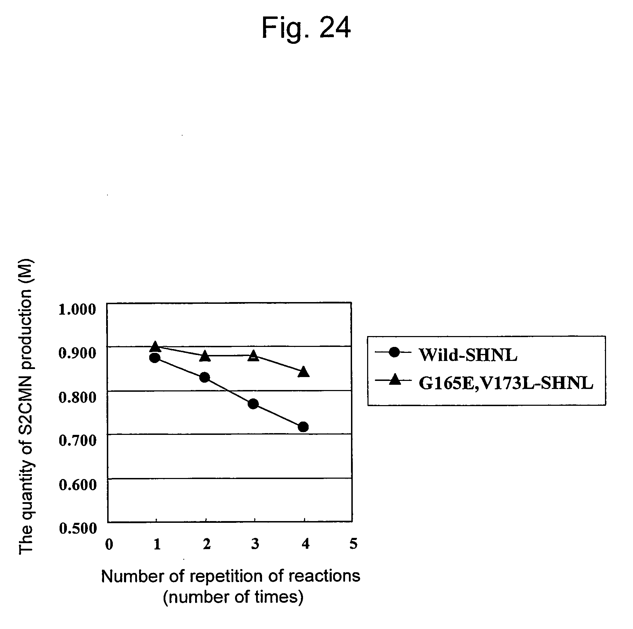

G165E,V173L-SHNL produced (S)-2-chloromandelonitrile at the same reaction rate as wild-SHNL in the first reaction. Accordingly, G165E,V173L-SHNL was found to have the capacity for producing optically active cyanohydrin, which is substantially the same as that of wild-SHNL in terms of productivity. As the number of repetition of reactions increased, enzyme activity and reaction rate of both strains were lowered. However, the degree of lowering for G165E,V173L-SHNL was apparently milder than that for wild-SHNL (Fig. 24). Thus, G165E,V173L-SHNL was found to have improved stability in the enzyme reaction system as well as improved heat resistance.

### Reference Example 20: Construction of mutant enzyme in which amino acid 163 (Thr) had been mutated

Position 163 in the amino acid sequence of SHNL was filled with various types of amino acids, and influences thereof on the thermostability of SHNL were examined.

### 1) Mutagenesis

In the same manner as in Reference Example 8, the QuikChange XL Site-Directed Mutagenesis Kit (Stratagene) was employed. An extension reaction was carried out using 10 ng of pET21a/SHNL-Wild as a template and primers as shown in SEQ ID NO: 23 and in SEQ ID NO: 24. Subsequently, the resulting reaction product was digested with the *Dpn*l restriction enzyme included in the kit.
tgaaaacctg ttcaccaaat gcnnngatgg cgaatatgaa ctggc (SEQ ID NO: 23)
gccagttcat attcgccatc nnngcatttg gtgaacaggt tttca (SEQ ID NO: 24)

### 2) Transformation

The resulting reaction product processed with restriction enzymes was applied to XL10-Gold competent cells included in the kit for transformation and then cultured on an LB (Amp) plate. The resulting colonies formed on the plate were resuspended in LB (Amp) liquid medium to isolate the pET21a/SHNL-SDThr163NNN plasmid. This plasmid was applied to BL21 (DE3) competent cells (Novagen) for transformation to prepare a plurality of BL21(DE3)/pET21a/SHNL-SDThr163NNN strains.

### 3) Selection of mutant SHNL

The prepared E. *coli* BL21(DE3)/pET21a/SHNL-SD Thr163NNN strains were cultured in the same manner as in Reference Example 2. The resulting culture broths were employed to select mutants having improved heat resistance in accordance with the method of Reference Example 1. As a result, mutants, i.e., BL21(DE3)/pET21a/SHNL-SD163-1b5, BL21(DE3)/pET21a/SHNL-SD163-1f5, and BL21(DE3)/pET21a/SHNL-SD163-1f7, were found to be still active after heating. Subsequently, colony PCR was carried out using the following primers and the selected strains as templates. Further, sequencing was carried out using the resulting PCR product as a template and the same primers.
Forward primer:
5'-TGAAAACCTGTTCACCAAATGCNNNGATGGCGAATATGAACTGGC-3' (SEQ ID NO: 25)
Reverse primer:
5'-GCCAGTTCATATTCGCCATCNNNGCATTTGGTGAACAGGTTTTCA-3' (SEQ ID NO: 26)

The analysis of the reaction product demonstrated that SD163-1b5-SHNL had a nucleotide sequence derived from the nucleotide sequence as shown in SEQ ID NO: 1 by substitution of amino acids 487 to 489 with GAT. Accordingly, it was verified that SD163-1b5-SHNL was a mutant SHNL having an amino acid derived from the amino acid sequence of wild-SHNL by substitution of threonine as amino acid 163 with aspartic acid. Similarly, SD163-1f5-SHNL had a nucleotide sequence derived from the nucleotide sequence as shown in SEQ ID NO: 1 by modification of amino acids 487 to 489 to GAA. Accordingly, it was verified that SD163-1f5-SHNL was a mutant SHNL having an amino acid sequence derived from the amino acid sequence of wild-SHNL by substitution of threonine as amino acid 163 with glutamic acid. Further, SHNL-SD163-1f7 has a nucleotide sequence derived from the nucleotide sequence as shown in SEQ ID NO: 1 by modification of amino acids 487 to 489 to TCT. Accordingly, it was verified that SD163-1f7 SHNL was a mutant SHNL having an amino acid sequence derived from the amino acid sequence of wild-SHNL by substitution of threonine as amino acid 163 with serine.

Hereafter, SD163-1b5-SHNL is referred to as "T165D-SHNL," SD163-1f5-SHNL is referred to as "T163E-SHNL," and SD163-1f7-SHNL is referred to as "T163S-SHNL."

### Reference Example 21: Thermostability of mutant enzymes, i.e., T163D-SHNL, T163E-SHNL, and T163S-SI-INL

### 1. Method of experimentation

### 1) Preparation of enzyme solution

The *E. coli* strains, BL21(DE3)/pET21a/SHNL-T163D, BL21(DE3)/pET21a/SHNL-T163E, and BL21(DE3)/pET21a/SHNL-T163S, which had been constructed in Reference Example 20, were cultured in the same manner as in Reference Example 2 to prepare enzyme solutions. Further, the prepared enzyme solutions were diluted with bovine serum albumin and a 0.2 M sodium citrate buffer, BL21(DE3)/pET21a/SHNL-T163D and BL21(DE3)/pET21a/SHNL-T163E were adjusted to have an activity level of 70 U/ml and a protein concentration of 7 mg/ml. BL21(DE3)/pET21a/SHNL-T163S was adjusted to have an activity level of 70 U/ml and a protein concentration of 14 mg/ml. As a control, BL21(DE3)/pET21a/SHNL-Wild that had been adjusted to the same concentration was employed.

### 2) Heat treatment of enzyme solution

The enzyme solutions were heated to 50°C to 70°C in the same manner as in Reference Example 2. The enzyme solutions were centrifuged 30 minutes after initiating heating, and the supernatant was subjected to assay of residual activity in relation to enzyme activity before heating.

### 2. Result of experimentation

T163S-SHNL exhibited significantly improved thermostability compared with that of wild-SHNL (Fig. 12). Also, thermostability of T163D-SHNL and that of T163E-SHNL at 60°C were superior to those of wild-SHNL. Thus, it was found that substitution of threonine as amino acid 163 that constitutes helix D3' with aspartic acid, glutamic acid, or serine could improve thermostability of SHNL.

All publications, patents, and patent applications cited herein are incorporated herein by reference in their entirety.

### Industrial Applicability

The acid-resistant modified SHNL according to the present invention has improved acid resistance compared with wild-type SHNL. Accordingly, the acid-resistant modified SHNL according to the present invention can react under acidic conditions, and it can synthesize highly-purified optically active cyanohydrin with high efficiency while suppressing competitive racemization. Therefore, the acid-resistant modified SHNL according to the present invention is a very useful enzyme for producing optically active cyanohydrin at industrial levels.

### Free Text of Sequence Listing

SEQ ID NO: 5: description of artificial sequence: primer
SEQ ID NO: 6: description of artificial sequence: primer
SEQ ID NO: 7: description of artificial sequence: primer
SEQ ID NO: 8: description of artificial sequence: primer
SEQ ID NO: 9: description of artificial sequence: primer
SEQ ID NO: 10: description of artificial sequence: primer
SEQ ID NO: 11: description of artificial sequence: primer
SEQ ID NO: 12: description of artificial sequence: primer
SEQ ID NO: 13: description of artificial sequence: primer
SEQ ID NO: 14: description of artificial sequence: primer
SEQ ID NO: 15: description of artificial sequence: primer
SEQ ID NO: 16: description of artificial sequence: primer
SEQ ID NO: 17: description of artificial sequence: primer
SEQ ID NO: 18: description of artificial sequence: primer
SEQ ID NO: 19: description of artificial sequence: primer
SEQ ID NO: 20: description of artificial sequence: primer
SEQ ID NO: 21: description of artificial sequence: primer
SEQ ID NO: 22: description of artificial sequence: primer
SEQ ID NO: 23: description of artificial sequence: primer
SEQ ID NO: 24: description of artificial sequence: primer
SEQ ID NO: 25: description of artificial sequence: primer
SEQ ID NO: 26: description of artificial sequence: primer

## Claims

1. Modified S-hydroxynitrile lyase (SHNL), which is obtained by substituting at least one amino acid selected from amino acids 36, 140, and 209 in the amino acid sequence (SEQ ID NO: 2) of wild-type S-hydroxynitrile lyase derived from cassava (*Manihot esculenta*) with another amino acid or modified S-hydroxynitrile lyase, which is obtained by substituting at least one amino acid selected from amino acids 36, 139, and 208 in the amino acid sequence (SEQ ID NO: 3) of wild-type S-hydroxynitrile lyase derived from the Pará rubber tree (*Hevea brasiliensis*) with another amino acid.

2. Modified S-hydroxynitrile lyase comprising at least one amino acid substitution selected from the amino acid substitutions shown below in the amino acid sequence (SEQ ID NO: 2) of wild-type hydroxynitrile lyase derived from cassava (*Manihot esculenta*):
a) substitution of leucine 36 with methionine;
b) substitution of threonine 140 with isoleucine; and
c) substitution of lysine 209 with asparagine.

3. The modified S-hydroxynitrile lyase according to claim 2, which further comprises at least one amino acid substitution selected from the following amino acid substitutions:
a) substitution of lysine 21 with aspartic acid, glutamic acid, or asparagine;
b) substitution of glycine 165 with aspartic acid or glutamic acid;
c) substitution of valine 173 with leucine;
d) substitution of methionine 174 with leucine; and
e) substitution of threonine 163 with aspartic acid, glutamic acid, or serine.

4. DNA that encodes the amino acid sequence of the modified S-hydroxynitrile lyase according to any one of claims 1 to 3.

5. A method for producing modified S-hydroxynitrile lyase comprising culturing a host cell into which the DNA according to claim 4 has been introduced and recovering a protein having S-hydroxynitrile lyase activity from the resulting culture product.

6. A method for producing optically active cyanohydrin comprising allowing the modified S-hydroxynitrile lyase according to any one of claims 1 to 3 to react with a carbonyl compound and cyanide.


SEQUENCE LISTING

<110> NIPPON SHOKUBAI CO., LTD.
      National Institute of Technology and Evaluation

<120> Novel acid-resistant S-hydroxynitrile lyase

<130> PH-2925-PCT

<150> JP 2005-285049
<151> 2005-09-29

<160> 26

<170> PatentIn Ver. 2.1

<210> 1
<211> 777
<212> DNA
<213> Manihot esculenta

<220>
<221> CDS
<222> (1)..(777)

<400> 1
atg gtt act gca cac ttc gtt ctg att cac acc att tgt cac ggc gca    48
Met Val Thr Ala His Phe Val Leu Ile His Thr Ile Cys His Gly Ala
  1               5                  10                  15

tgg att tgg cac aaa ctg aaa ccg gcc ctg gaa cgt gct ggc cac aaa    96
Trp Ile Trp His Lys Leu Lys Pro Ala Leu Glu Arg Ala Gly His Lys
             20                  25                  30

gtt act gca ctg gac atg gca gcc agt ggc att gac ccg cgt caa att    144
Val Thr Ala Leu Asp Met Ala Ala Ser Gly Ile Asp Pro Arg Gln Ile
         35                  40                  45

gaa cag atc aac tct ttc gat gaa tac tct gaa ccg ctg ctg act ttc    192
Glu Gln Ile Asn Ser Phe Asp Glu Tyr Ser Glu Pro Leu Leu Thr Phe
     50                  55                  60

ctg gaa aaa ctg ccg caa ggc gaa aag gtt atc att gtt ggt gaa agc    240
Leu Glu Lys Leu Pro Gln Gly Glu Lys Val Ile Ile Val Gly Glu Ser
 65                  70                  75                  80

tgt gca ggc ctg aac att gct att gct gct gat cgt tac gtt gac aaa 288
Cys Ala Gly Leu Asn Ile Ala Ile Ala Ala Asp Arg Tyr Val Asp Lys
                85                  90                  95

att gca gct ggc gtt ttc cac aac tcc ctg ctg ccg gac acc gtt cac 336
Ile Ala Ala Gly Val Phe His Asn Ser Leu Leu Pro Asp Thr Val His
            100                 105                 110

agc ccg tct tac act gtt gaa aag ctg ctg gaa tcg ttc ccg gac tgg 384
Ser Pro Ser Tyr Thr Val Glu Lys Leu Leu Glu Ser Phe Pro Asp Trp
        115                 120                 125

cgt gac aca gaa tat ttc acg ttc acc aac atc act ggc gaa acc atc 432
Arg Asp Thr Glu Tyr Phe Thr Phe Thr Asn Ile Thr Gly Glu Thr Ile
    130                 135                 140

act acc atg aaa ctg ggt ttc gtt ctg ctg cgt gaa aac ctg ttc acc 480
Thr Thr Met Lys Leu Gly Phe Val Leu Leu Arg Glu Asn Leu Phe Thr
145                 150                 155                 160

aaa tgc act gat ggc gaa tat gaa ctg gca aaa atg gtt atg cgc aag 528
Lys Cys Thr Asp Gly Glu Tyr Glu Leu Ala Lys Met Val Met Arg Lys
                165                 170                 175

ggc tct ctg ttc caa aac gtt ctg gct cag cgt ccg aag ttc act gaa 576
Gly Ser Leu Phe Gln Asn Val Leu Ala Gln Arg Pro Lys Phe Thr Glu
            180                 185                 190

aaa ggc tac ggc tct atc aag aaa gtt tat att tgg acc gat caa gac 624
Lys Gly Tyr Gly Ser Ile Lys Lys Val Tyr Ile Trp Thr Asp Gln Asp
        195                 200                 205

aaa ata ttc ctg ccg gac ttc caa cgc tgg caa att gca aac tac aaa 672
Lys Ile Phe Leu Pro Asp Phe Gln Arg Trp Gln Ile Ala Asn Tyr Lys
    210                 215                 220

ccg gac aag gtt tat cag gtt caa ggc ggc gat cac aag ctg cag ctg 720
Pro Asp Lys Val Tyr Gln Val Gln Gly Gly Asp His Lys Leu Gln Leu
225                 230                 235                 240

aca aaa act gaa gaa gta gct cac att ctg caa gaa gtt gct gat gca 768
Thr Lys Thr Glu Glu Val Ala His Ile Leu Gln Glu Val Ala Asp Ala
                245                 250                 255

tac gct taa 777

Tyr Ala

<210> 2
<211> 258
<212> PRT
<213> Manihot esculenta

<400> 2
Met Val Thr Ala His Phe Val Leu Ile His Thr Ile Cys His Gly Ala
1               5                   10                  15
Trp Ile Trp His Lys Leu Lys Pro Ala Leu Glu Arg Ala Gly His Lys
            20                  25                  30
Val Thr Ala Leu Asp Met Ala Ala Ser Gly Ile Asp Pro Arg Gln Ile
        35                  40                  45
Glu Gln Ile Asn Ser Phe Asp Glu Tyr Ser Glu Pro Leu Leu Thr Phe
    50                  55                  60
Leu Glu Lys Leu Pro Gln Gly Glu Lys Val Ile Ile Val Gly Glu Ser
65                  70                  75                  80
Cys Ala Gly Leu Asn Ile Ala Ile Ala Ala Asp Arg Tyr Val Asp Lys
                85                  90                  95
Ile Ala Ala Gly Val Phe His Asn Ser Leu Leu Pro Asp Thr Val His
            100                 105                 110
Ser Pro Ser Tyr Thr Val Glu Lys Leu Leu Glu Ser Phe Pro Asp Trp
        115                 120                 125
Arg Asp Thr Glu Tyr Phe Thr Phe Thr Asn Ile Thr Gly Glu Thr Ile
    130                 135                 140
Thr Thr Met Lys Leu Gly Phe Val Leu Leu Arg Glu Asn Leu Phe Thr
145                 150                 155                 160
Lys Cys Thr Asp Gly Glu Tyr Glu Leu Ala Lys Met Val Met Arg Lys
                165                 170                 175
Gly Ser Leu Phe Gln Asn Val Leu Ala Gln Arg Pro Lys Phe Thr Glu
            180                 185                 190
Lys Gly Tyr Gly Ser Ile Lys Lys Val Tyr Ile Trp Thr Asp Gln Asp
        195                 200                 205
Lys Ile Phe Leu Pro Asp Phe Gln Arg Trp Gln Ile Ala Asn Tyr Lys
    210                 215                 220
Pro Asp Lys Val Tyr Gln Val Gln Gly Gly Asp His Lys Leu Gln Leu
225                 230                 235                 240
Thr Lys Thr Glu Glu Val Ala His Ile Leu Gln Glu Val Ala Asp Ala
                245                 250                 255
Tyr Ala

<210> 3

<211> 774
<212> DNA
<213> Manihot esculenta

<220>
<221> CDS
<222> (1)..(774)

<400> 3
atg gca ttc gct cat ttt gtt ctt att cat acc ata tgc cac ggt gca     48
Met Ala Phe Ala His Phe Val Leu Ile His Thr Ile Cys His Gly Ala
1               5                   10                  15

tgg att tgg cac aag ctc aaa ccc ctc ctt gag gca ctt ggc cac aag     96
Trp Ile Trp His Lys Leu Lys Pro Leu Leu Glu Ala Leu Gly His Lys
            20                  25                  30

gtt act gca ctg gac ctt gca gca agc ggc gtt gac cca agg caa att     144
Val Thr Ala Leu Asp Leu Ala Ala Ser Gly Val Asp Pro Arg Gln Ile
        35                  40                  45

gag gag att ggc tca ttt gat gag tat tct gaa ccc ttg ttg acg ttc     192
Glu Glu Ile Gly Ser Phe Asp Glu Tyr Ser Glu Pro Leu Leu Thr Phe
    50                  55                  60

ttg gag gca ctc cct cca ggg gaa aag gtg att ctg gtt ggc gag agc     240
Leu Glu Ala Leu Pro Pro Gly Glu Lys Val Ile Leu Val Gly Glu Ser
65                  70                  75                  80

tgt gga gga ctc aat ata gca att gct gct gat aaa tac tgt gaa aag     288
Cys Gly Gly Leu Asn Ile Ala Ile Ala Ala Asp Lys Tyr Cys Glu Lys
                85                  90                  95

att gca gct gct gtt ttc cac aat tca gta ttg cca gac acc gag cac     336
Ile Ala Ala Ala Val Phe His Asn Ser Val Leu Pro Asp Thr Glu His
            100                 105                 110

tgc cca tct tac gtc gtg gat aag ctc atg gag gtg ttt ccc gac tgg     384
Cys Pro Ser Tyr Val Val Asp Lys Leu Met Glu Val Phe Pro Asp Trp
        115                 120                 125

aaa gac acc acg tat ttt acg tac act aaa gat ggc aag gag ata act     432
Lys Asp Thr Thr Tyr Phe Thr Tyr Thr Lys Asp Gly Lys Glu Ile Thr
    130                 135                 140

gga ttg aaa ctg ggc ttc acg ctt ctg agg gaa aat tta tat acc ctt     480

Gly Leu Lys Leu Gly Phe Thr Leu Leu Arg Glu Asn Leu Tyr Thr Leu
145                 150                 155                 160

tgc ggt cct gag gaa tat gaa ctg gcg aag atg ttg aca agg aag gga     528
Cys Gly Pro Glu Glu Tyr Glu Leu Ala Lys Met Leu Thr Arg Lys Gly
                165                 170                 175

tca tta ttt caa aat att tta gct aag cga cca ttc ttc act aag gaa     576
Ser Leu Phe Gln Asn Ile Leu Ala Lys Arg Pro Phe Phe Thr Lys Glu
            180                 185                 190

ggt tac gga tcg att aag aaa att tat gtg tgg acc gac caa gac gaa     624
Gly Tyr Gly Ser Ile Lys Lys Ile Tyr Val Trp Thr Asp Gln Asp Glu
        195                 200                 205

ata ttt tta cct gaa ttt caa ctc tgg caa ata gaa aac tat aaa cca     672
Ile Phe Leu Pro Glu Phe Gln Leu Trp Gln Ile Glu Asn Tyr Lys Pro
    210                 215                 220

gac aag gtt tat aag gtc gaa ggt gga gat cat aaa ttg cag ctt aca     720
Asp Lys Val Tyr Lys Val Glu Gly Gly Asp His Lys Leu Gln Leu Thr
225                 230                 235                 240

aag act aag gag atc gct gaa att ctc caa gag gtg gct gat acc tat     768
Lys Thr Lys Glu Ile Ala Glu Ile Leu Gln Glu Val Ala Asp Thr Tyr
                245                 250                 255

aat tga                                                             774
Asn

<210> 4
<211> 257
<212> PRT
<213> Manihot esculenta

<400> 4
Met Ala Phe Ala His Phe Val Leu Ile His Thr Ile Cys His Gly Ala
  1               5                  10                  15
Trp Ile Trp His Lys Leu Lys Pro Leu Leu Glu Ala Leu Gly His Lys
             20                  25                  30
Val Thr Ala Leu Asp Leu Ala Ala Ser Gly Val Asp Pro Arg Gln Ile
         35                  40                  45
Glu Glu Ile Gly Ser Phe Asp Glu Tyr Ser Glu Pro Leu Leu Thr Phe
     50                  55                  60
Leu Glu Ala Leu Pro Pro Gly Glu Lys Val Ile Leu Val Gly Glu Ser

65                  70                  75                  80
Cys Gly Gly Leu Asn Ile Ala Ile Ala Ala Asp Lys Tyr Cys Glu Lys
                85                  90                  95
Ile Ala Ala Ala Val Phe His Asn Ser Val Leu Pro Asp Thr Glu His
            100                 105                 110
Cys Pro Ser Tyr Val Val Asp Lys Leu Met Glu Val Phe Pro Asp Trp
        115                 120                 125
Lys Asp Thr Thr Tyr Phe Thr Tyr Thr Lys Asp Gly Lys Glu Ile Thr
    130                 135                 140
Gly Leu Lys Leu Gly Phe Thr Leu Leu Arg Glu Asn Leu Tyr Thr Leu
145                 150                 155                 160
Cys Gly Pro Glu Glu Tyr Glu Leu Ala Lys Met Leu Thr Arg Lys Gly
                165                 170                 175
Ser Leu Phe Gln Asn Ile Leu Ala Lys Arg Pro Phe Phe Thr Lys Glu
            180                 185                 190
Gly Tyr Gly Ser Ile Lys Lys Ile Tyr Val Trp Thr Asp Gln Asp Glu
        195                 200                 205
Ile Phe Leu Pro Glu Phe Gln Leu Trp Gln Ile Glu Asn Tyr Lys Pro
    210                 215                 220
Asp Lys Val Tyr Lys Val Glu Gly Gly Asp His Lys Leu Gln Leu Thr
225                 230                 235                 240
Lys Thr Lys Glu Ile Ala Glu Ile Leu Gln Glu Val Ala Asp Thr Tyr
                245                 250                 255
Asn

<210> 5
<211> 39
<212> DNA
<213> Artificial Sequence

<220>
<223> Description of Artificial Sequence: primer

<400> 5
ggccacaaag ttactgcaat ggacatggca gccagtggc                          39

<210> 6
<211> 39
<212> DNA
<213> Artificial Sequence

<220>
<223> Description of Artificial Sequence: primer

<400> 6
gccactggct gccatgtcca ttgcagtaac tttgtggcc                                39

<210> 7
<211> 40
<212> DNA
<213> Artificial Sequence

<220>
<223> Description of Artificial Sequence: primer

<400> 7
cacgttcacc aacatcatcg gcgaaaccat cactaccatg                               40

<210> 8
<211> 40
<212> DNA
<213> Artificial Sequence

<220>
<223> Description of Artificial Sequence: primer

<400> 8
catggtagtg atggtttcgc cgatgatgtt ggtgaacgtg                               40

<210> 9
<211> 45
<212> DNA
<213> Artificial Sequence

<220>
<223> Description of Artificial Sequence: primer

<400> 9
atttggaccg atcaagacaa catattcctg ccggacttcc aacgc                         45

<210> 10
<211> 45
<212> DNA
<213> Artificial Sequence

<220>
<223> Description of Artificial Sequence: primer

<400> 10
gcgttggaag tccggcagga atatgttgtc ttgatcggtc caaat 45

<210> 11
<211> 39
<212> DNA
<213> Artificial Sequence

<220>
<223> Description of Artificial Sequence: primer

<400> 11
ggggaattca tggttactgc acacttcgtt ctgattcac 39

<210> 12
<211> 39
<212> DNA
<213> Artificial Sequence

<220>
<223> Description of Artificial Sequence: primer

<400> 12
gggaagcttt taagcgtatg catcagcaac ttcttgcag 39

<210> 13
<211> 44
<212> DNA
<213> Artificial Sequence

<220>
<223> Description of Artificial Sequence: primer

<400> 13
gggggggggc atatggttac tgcacacttc gttctgattc acac 44

<210> 14
<211> 39
<212> DNA

<213> Artificial Sequence

<220>
<223> Description of Artificial Sequence: primer

<400> 14
gggggatcct taagcgtatg catcagcaac ttcttgcag                    39


<210> 15
<211> 54
<212> DNA
<213> Artificial Sequence

<220>
<223> Description of Artificial Sequence: primer

<400> 15
cgtgaaaacc tgttcaccaa atgcactgat gaagaatatg aactggcaaa aatg        54


<210> 16
<211> 54
<212> DNA
<213> Artificial Sequence

<220>
<223> Description of Artificial Sequence: primer

<400> 16
catttttgcc agttcatatt cttcatcagt gcatttggtg aacaggtttt cacg        54


<210> 17
<211> 45
<212> DNA
<213> Artificial Sequence

<220>
<223> Description of Artificial Sequence: primer

<400> 17
ggcgaatatg aactggcaaa aatgnnnatg cgcaagggct ctctg                  45


<210> 18

<211> 45
<212> DNA
<213> Artificial Sequence

<220>
<223> Description of Artificial Sequence: primer

<400> 18
cagagagccc ttgcgcatnn ncatttttgc cagttcatat tcgcc 45

<210> 19
<211> 39
<212> DNA
<213> Artificial Sequence

<220>
<223> Description of Artificial Sequence: primer

<400> 19
ggcgcatgga tttggcacnn nctgaaaccg gccctggaa 39

<210> 20
<211> 39
<212> DNA
<213> Artificial Sequence

<220>
<223> Description of Artificial Sequence: primer

<400> 20
ttccagggcc ggtttcagnn ngtgccaaat ccatgcgcc 39

<210> 21
<211> 42
<212> DNA
<213> Artificial Sequence

<220>
<223> Description of Artificial Sequence: primer

<400> 21
tatgaactgg caaaaatgct gctgcgcaag ggctctctgt tc 42

<210> 22
<211> 42
<212> DNA
<213> Artificial Sequence

<220>
<223> Description of Artificial Sequence: primer

<400> 22
gaacagagag cccttgcgca gcagcatttt tgccagttca ta                42

<210> 23
<211> 45
<212> DNA
<213> Artificial Sequence

<220>
<223> Description of Artificial Sequence: primer

<400> 23
tgaaaacctg ttcaccaaat gcnnngatgg cgaatatgaa ctggc             45

<210> 24
<211> 45
<212> DNA
<213> Artificial Sequence

<220>
<223> Description of Artificial Sequence: primer

<400> 24
gccagttcat attcgccatc nnngcatttg gtgaacaggt tttca             45

<210> 25
<211> 45
<212> DNA
<213> Artificial Sequence

<220>
<223> Description of Artificial Sequence: primer

<400> 25

tgaaaacctg ttcaccaaat gcnnngatgg cgaatatgaa ctggc 45

<210> 26
<211> 45
<212> DNA
<213> Artificial Sequence

<220>
<223> Description of Artificial Sequence: primer

<400> 26
gccagttcat attcgccatc nnngcatttg gtgaacaggt tttca 45